(19)

Europäisches
Patentamt

European
Patent Office

Office européen
des brevets

(11) **EP 3 650 556 A1**

(12) **EUROPEAN PATENT APPLICATION**

(43) Date of publication:
**13.05.2020  Bulletin 2020/20**

(51) Int Cl.:
*C12Q 1/6809* (2018.01)    *C12Q 1/6886* (2018.01)

(21) Application number: **18020578.3**

(22) Date of filing: **06.11.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Stichting Het Nederlands Kanker Instituut-
Antoni van Leeuwenhoek Ziekenhuis
1066 CX Amsterdam (NL)**
• **Koninklijke Nederlandse Akademie van
Wetenschappen
1011 JV  Amsterdam (NL)**

(72) Inventors:
• **SEINSTRA, Danielle
1066CX AMSTERDAM (NL)**
• **VAN OUDENAARDEN, Alexander
1011JV Amsterdam (NL)**
• **RHEENEN, Jacobus Emiel
1066CX Amsterdam (NL)**
• **KESTER, Lennert Anne
1066CX Amsterdam (NL)**

(74) Representative: **Algemeen Octrooi- en
Merkenbureau B.V.
P.O. Box 645
5600 AP Eindhoven (NL)**

(54) **METHOD FOR DETERMINING CELLULAR COMPOSITION OF A TUMOR**

(57)    The invention relates to methods for determining the cellular composition of a tumor based on bulk mRNA sequencing, and to methods for constructing a reference library comprising single-cell sequencing data for use in the method for determining the cellular composition.

EP 3 650 556 A1

**Description**

<u>Field of the invention</u>

The invention relates to a method for determining the cellular composition of a tumor of a subject based on bulk mRNA sequencing. The invention further relates to making a prediction for a subject suffering from cancer based on the cellular composition of the tumor obtained from said subject. The invention also relates to a method for constructing a reference library comprising single-cell sequencing data suitable for use in the method for determining the cellular composition of a tumor of a patient. The invention further relates to reference libraries suitable for use in the method of the invention.

<u>Background of the invention</u>

Cancer is a major cause of death. Tumors from different patients can have distinct pathological features and, more importantly, different sensitivities to therapeutics. To optimize treatment strategies, a large effort has been invested into classifying these tumors based on histological and cytological features such as growth patterns, and more recently into molecular and intrinsic subtypes of cancer such as expression profiles or genomic alterations.

Unfortunately, classification of tumors into subtypes does not always correctly predict the response to cytotoxic and targeted therapy. It is widely debated whether non-responding tumor cells, already present in the tumor, are positively selected for upon treatment, and/or whether therapy-resistance is induced by the therapy, for example by changing tumor-supporting stromal/immune cells in the microenvironment.

Until now, characterization of potential co-existing sub-clones of tumorigenic cells and tumor-supporting non-cancer (non-tumorigenic) cells within a tumor has been challenging, even when analyzing biopsies from different sites of the tumor. First, non-cancer cells are not necessarily genetically aberrant, so DNA sequencing techniques cannot be used to characterize this population of cells. Second, RNA sequencing of bulk-tissue (e.g. biopsies) obscures the RNA signatures of less abundant co-existing clones (herein referred to as sub-types or sub-clones) and other distinct cell populations (e.g. cancer cells versus non-cancer stromal/immune cells). Therefore, the most prevalent cancer cell clone and stromal components in bulk measurements may potentially disguise information on smaller sub-populations of cancer and non-cancer cells that are important for therapy responses.

These limitations could be overcome by recent advances in single-cell mRNA sequencing, which have for example allowed the unbiased analysis of cellular diversity in melanoma, glioblastoma and colorectal carcinoma (Tirosh et al. 2016a, Tirosh et al. 2016b, Li et al. 2017).

Single-cell mRNA sequencing can provide insight in the cellular composition of a tumor. The presence or relative abundance of different non-tumor cell types can be distinguished with this technique, moreover single-cell mRNA sequencing is sensitive enough to distinguish between different sub-clones of tumor cells. This information can be used for example in predicting response to a specific therapy, survival rates, a treatment strategy and many more (see Patel et al. and Tirosh et al. 2016a, Li et al. 2017).

Although single-cell mRNA sequencing is a very potent tool to determine the cellular composition of a tumor, its use in diagnostics is impractical, due to, among others, the requirements of specialized equipment, highly trained personnel to perform the analysis, high cost and long processing time. Therefore, alternative methods for determining the cellular composition of a tumor which are more feasible as a diagnostic tool are desirable. In particular there is need for methods that do not suffer from these drawbacks but provide similar opportunities as single-cell mRNA sequencing.

To overcome these problems, among others, the present invention aims to use a reference library based on single-cell mRNA sequencing to characterize the cellular composition of tumors from cancer patients. This reference library holds important information on less prevalent, but clinically relevant, cellular components of the tumor, such as tumorigenic cell sub-types and non-tumorigenic cells. The invention further aims to compare bulk mRNA sequencing data obtained from a subject with the reference library to identify the cellular composition of individual tumors obtained from a subject based on bulk mRNA sequencing data, since mRNA sequencing of biopsies is becoming more common in cancer diagnosis. In a preferred embodiment, such comparing is done using a computational algorithm. By performing such comparison, for example by using a computational algorithm, identification of the cellular composition of a tumor can be determined, providing novel insights in therapeutic sensitivities and survival, which further improve classification and treatment selection, and assists clinical decisions making.

<u>Summary of the invention</u>

In an aspect, the invention relates to a method for determining the cellular composition of a tumor from a subject suffering from cancer, the method comprising the steps of:

- a) performing bulk mRNA sequencing on tumor material obtained from the subject in order to obtain bulk sequencing data,
- b) comparing the bulk mRNA sequencing data of step a) with a reference library comprising clustered sequencing data in order to determine the relative contribution of each cluster comprised in the clustered sequencing data to the bulk sequencing data obtained from the tumor material obtained from the subject, and
- c) determining the cellular composition of the tumor material obtained from the subject based on the relative contribution of each cluster comprised in the clustered sequencing data to the bulk sequencing data.

In an aspect, the invention relates to a method for preparing a reference library, the method comprising the following steps:

- i) performing single-cell mRNA sequencing on individual cells isolated from tumor material obtained from at least one reference patient in order to obtain a sequencing data set for each sequenced cell,
- ii) generating a clustering of the sequenced single cells based on the sequencing data sets of step i) in order to obtain a reference library comprising clustered sequencing data sets.

In an aspect, the invention relates to a reference library obtainable by the method of the invention.

Definitions

The term "gene" means a DNA sequence comprising a region (transcribed region), which is transcribed into a RNA molecule (e.g. a mRNA) in a cell, operably linked to suitable regulatory regions (e.g. a promoter or distal enhancer). A gene may thus comprise several operably linked sequences, such as a promoter, a 5' leader sequence comprising e.g. sequences involved in translation initiation, a (protein) coding region (cDNA or genomic DNA), introns, and a 3'non-translated sequence comprising e.g. transcription termination sites.

When reference is made to the "expression level" of a gene, the expression level is to be understood as the amount of RNA transcript that is transcribed from a gene, e.g., mRNA and/or the amount of protein that may be translated from an RNA transcript, i.e., the amount of gene product. Thus, the amount of gene product that may be translated from an mRNA transcript may also be measured to determine the expression level of a gene. Methods for determining protein levels are known in the art, and include, without limitation, ELISA, mass spectrometry, immunohistochemistry and Western blot.

The term "cancer" refers to the presence of cells possessing characteristics typical of cancer-causing cells, such as uncontrolled proliferation, immortality, metastatic potential, rapid growth and proliferation rate, and certain characteristic morphological features. Often, cancer cells will be in the form of a tumor.

"Breast cancer" is a type of cancer originating from breast tissue, most commonly from the inner lining of milk ducts or the lobules that supply the ducts with milk. Breast cancer occurs in humans and other mammals. While the overwhelming majority of human cases occur in women, male breast cancer can also occur.

The following examples are meant for illustrative purposes only, and not intended to limit the scope of the present invention in any way.

The term "treating" as used herein means reversing, alleviating, inhibiting the progress of, or preventing, either completely or partially, the growth of tumors, tumor metastases, or other cancer- causing or neoplastic cells in a patient. The term "treatment" refers to the act of treating. When speaking about a "method of treating", it is referred to a procedure or course of action that is designed to reduce or eliminate the number of cancer cells in an animal, or to alleviate the symptoms of a cancer. "A method of treating" cancer does not necessarily mean that the cancer cells will be eliminated, that the number of cells will be reduced, or that the symptoms of a cancer will be alleviated. Often, a method of treating cancer will be performed even with a low likelihood of success, but which, given the medical history and estimated survival expectancy of a patient, is nevertheless deemed an overall beneficial course of action.

The term "resistant" in the context of treatment of a tumor with a hormonal therapeutic means that the hormonal therapeutic is not likely to have an optimal effect on the tumor; i.e., the effect of the hormonal therapeutic is reduced or absent.

The term "reference", as used herein, refers to a reference value for comparative purposes. The reference value may be a predetermined value determined for a population with a known outcome of treatment with a hormonal therapeutic. For example, the reference value may be a pre-determined population median of subjects with good outcome, or may be a pre-determined population median of subjects with poor outcome.

In this document and in its claims, the verb "to comprise" and its conjugations is used in its non-limiting sense to mean that items following the word are included, but items not specifically mentioned are not excluded. In addition, the verb "to consist of may be replaced by "to consist essentially of" meaning that a composition may comprise additional component(s) than the ones specifically identified, or a method may comprise additional step(s) than the ones specifically identified, said additional component(s) or additional step(s) not altering the unique characteristics of the invention.

In addition, reference to an element by the indefinite article "a" or "an" does not exclude the possibility that more than one of the element is present, unless the context clearly requires that there be one and only one of the elements. The indefinite article "a" or "an" thus usually means "at least one".

Description of Figures

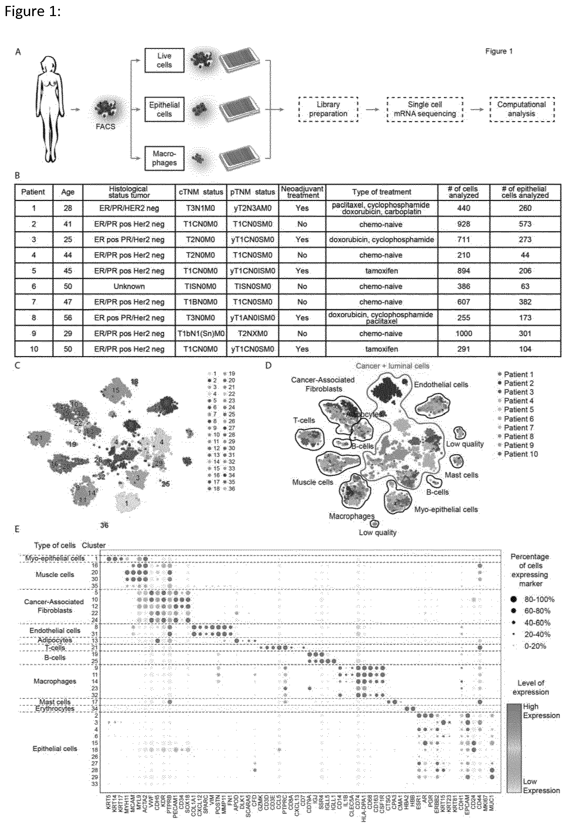

**Figure 1. Heterogeneity of breast tumors analyzed through single-cell mRNA sequencing**

Panel A shows a cartoon of the experimental design. Panel B shows patient and tumor characteristics. Panel C shows a t-Stochastic Neighbor Embedding (t-SNE) dimensional reduction of all cells on which the hierarchical RaceID clustering is projected. Each color and each number represent an individual RaceID cluster. Panel D shows the same t-SNE plot as in C and is highlighting the patient of origin for each single cell. Cell types are identified based on the RaceID clusters and for visual presentation highlighted by an outline. Panel E shows the expression of cell type specific marker genes across all RaceID clusters. Size of the dot indicates the percentage of cells within a cluster that express a marker gene, the color indicates the average level of expression in a cluster. The marker genes are examples of significantly enriched genes that can be found in each individual RaceID cluster.

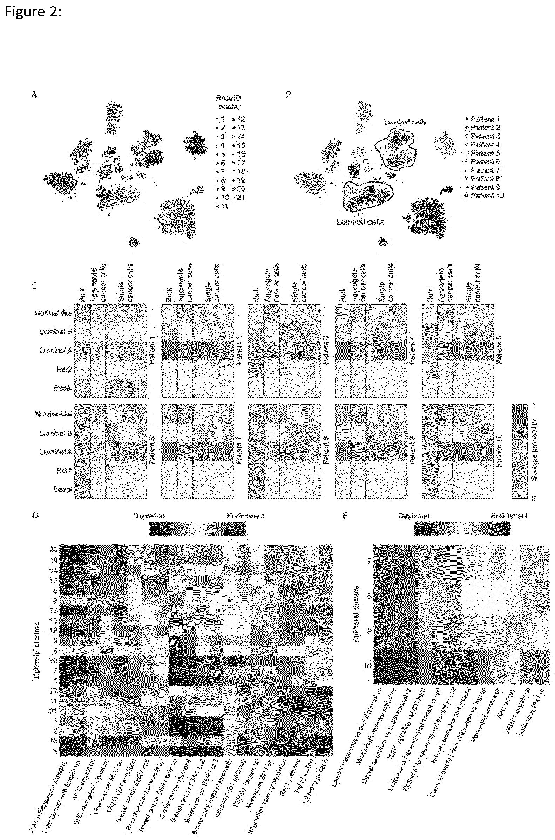

**Figure 2. Cancer cells in breast tumors are heterogeneous**

Panel A shows a t-Stochastic Neighbor Embedding (t-SNE) dimensional reduction of all epithelial cells on which the RaceID clustering is projected. Each color and each number represent an individual RaceID cluster. Panel B shows the same t-SNE plot as in A now highlighting the individual patients. The healthy luminal cells are indicated. Panel C shows the Pam50 molecular subtypes based on bulk mRNA sequencing and on single-cell mRNA sequencing. The color indicates the probability of a cell to belong to that particular subtype. Patients from which bulk mRNA sequencing could not be obtained are depicted in grey. For the Aggregate Cancer Cells PAM50 scores were calculated on the average expression profile of all cancer cells from a patient (excluding all microenvironmental and healthy luminal cells). Panel D shows a heatmap depicting significantly enriched of depleted MSigDB2 gene sets. Cluster specific significant enrichment or depletion of each gene set was calculated using a Wilcoxon test (Benjamini-Hochberg corrected p-value < 0.05) comparing the geneset enrichment in the single cells from each cluster to all single cells not belonging to that cluster. The y-axis indicates the RaceID cancer clusters and the x-axis indicates the geneset. The color indicates whether the geneset is enriched or depleted. Panel E shows a heatmap depicting genesets that are significantly different between cancer cluster 7, 8, 9 and cancer cluster 10. The y-axis indicates the cancer clusters and the x-axis indicates the geneset. The color indicates whether the geneset is enriched or depleted.

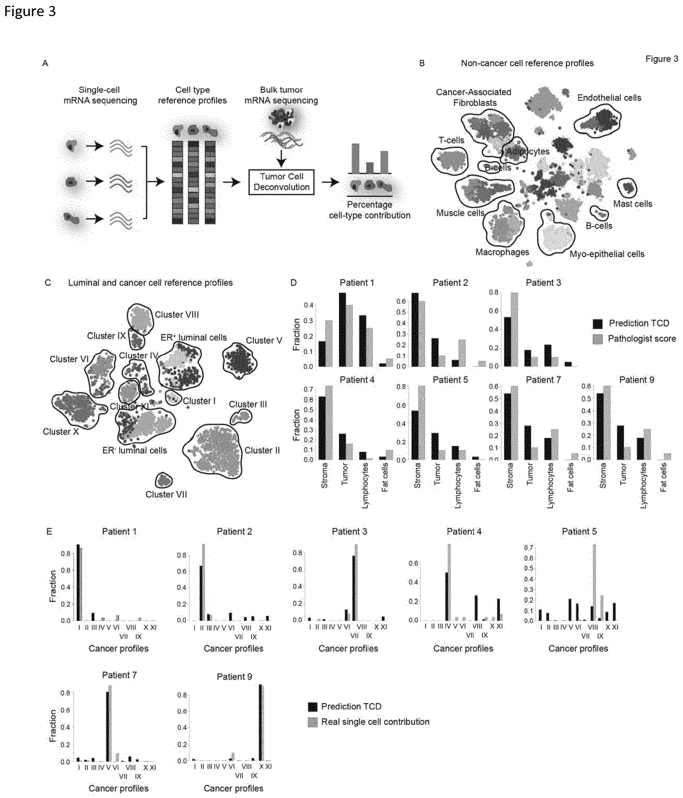

**Figure 3. Tumor Cell Deconvolution can accurately deconvolve breast cancer bulk mRNA sequencing**

Panel A shows a cartoon of the general principle of deconvolution. Panel B shows the same t-SNE plot as in Fig 1C, now highlighting the RaceID clusters that were used to construct the non-cancer reference profiles as used in TCD. Panel C shows the same t-SNE plot as in Fig 2A, now highlighting the RaceID clusters that were used to create the cancer cluster reference profiles as used in TCD. Panel D shows a series of barplots comparing the pathologist score of cell type abundances and the TCD prediction of cell type abundances. Panel E shows a series of barplots comparing the relative abundance of single cancer cells belonging to each of the cancer reference profiles (determined through single-cell mRNA sequencing) to the TCD cell type contribution predictions for the same cancer clusters based on the bulk mRNA sequencing.

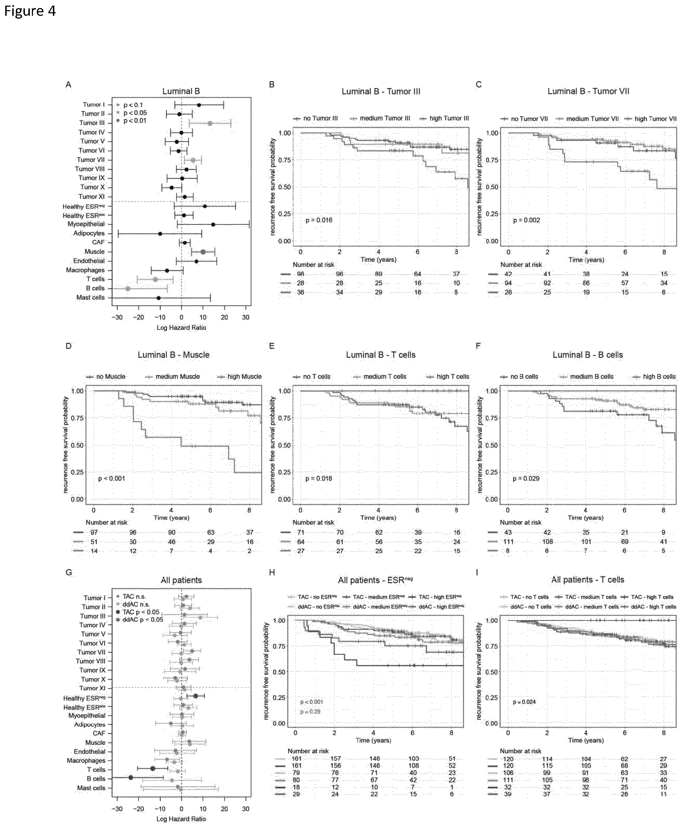

**Figure 4. Tumor Cell Deconvolution reveals new information on recurrence free survival upon treatment with ddAC or TAC**

This figure shows the Cox proportional hazard models and Kaplan-Meier curves for recurrence free survival probabilities for patient participating in the MATADOR trial. Panel A shows the Cox proportional hazard model for each of the cell types used in TCD for patients with luminal B tumors. Significance is indicated with black (not significant), green (fdr corrected p-value < 0.1), orange (fdr corrected p-value < 0.05) and or red (fdr corrected p-value < 0.01). In all Kaplan-Meier plots the numbers underneath the curve indicate the number of patients at risk at a certain time-point in that specific contribution group. The x-axis depicts time in years, the y-axis depicts the recurrence free survival probability. Panels B-E shows the survival probabilities for patients with low (< mean -1SD), medium (mean +/- 1SD) or high (>mean + 1SD) contribution of tumor III, tumor VII, muscle, T cells and B cells respectively in patients with luminal B tumors. Panel G shows the Cox proportional hazard model for each of the cell types used in TCD across all 528 patients, stratified by treatment. Significance is indicated with light-blue and light-red, not significant TAC and not significant ddAC respectively or dark-blue and dark-red, TAC fdr corrected p-value < 0.05 and ddAC fdr corrected p-value < 0.05. Panels H and I show the survival probabilities for patients treated with either TAC or ddAC with low, medium of high contribution of ESRneg or T cells respectively. P-values in panel H are Log Rank Test p-values for the TAC treated patients (red) and ddAC treated patients (blue). P-value in panel I is the Log Rank Test p-value between TAC treated patients with high T cell contribution and ddAC patients with high T cell contribution.

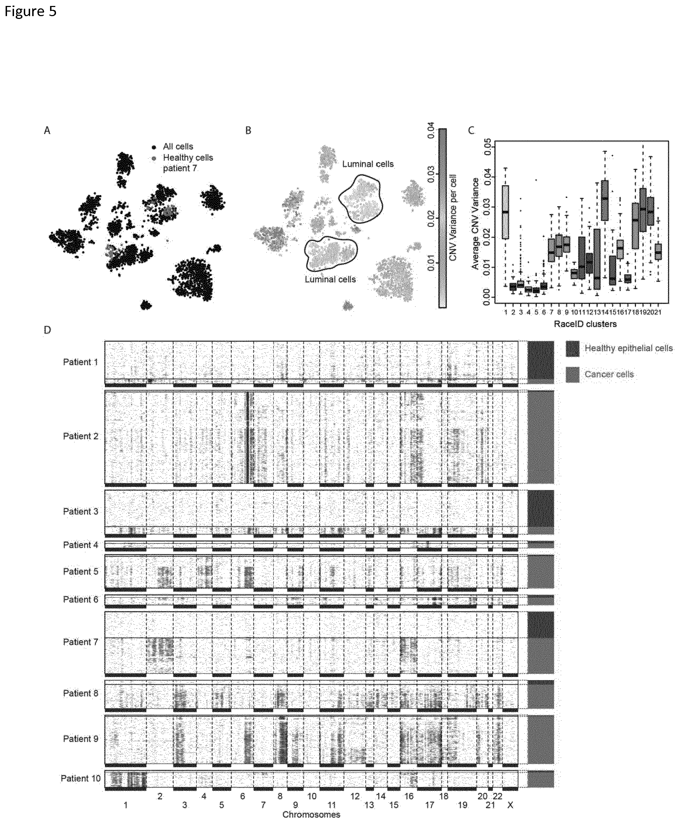

**Figure 5. Identification of healthy luminal cells**

Panel A shows the same t-SNE plot as in figure 2A, the healthy epithelial cells from the contralateral breast of patient 7 are highlighted in red. Panel B shows the same t-SNE plot depicting the variance of the copy number variation profiles across the genome for each single cell. Low variance indicates that the genome is stable and does not have chromosomal aberrations. Panel C shows the copy number profile variance per RaceID cluster. Clusters 2-6 show a low copy number profile variance and are therefore the untransformed luminal epithelial cells. Panel D shows the copy number profiles per cell for each patient. The chromosomes are aligned on the x-axis. The color bar on the right indicates the healthy epithelial cells (blue) and the cancer cells (red).

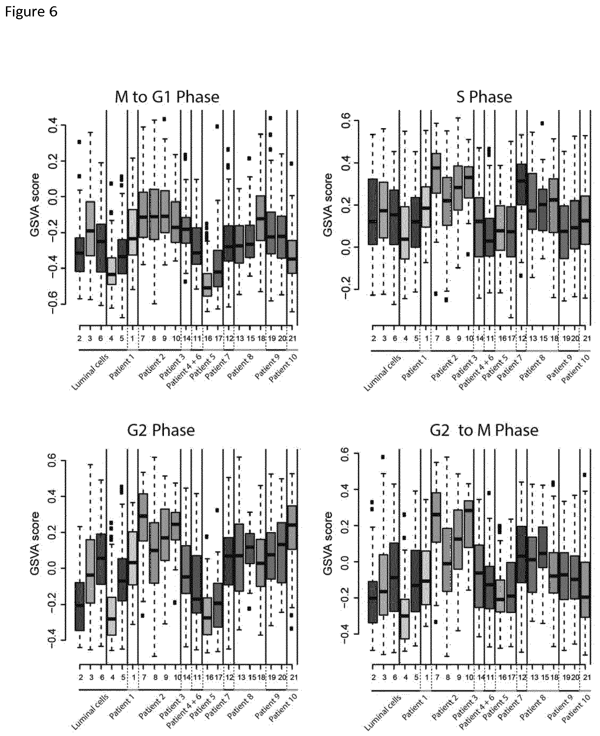

**Figure 6. Cell cycle specific genesets per cancer cluster**

Shown are the GSVA enrichment scores for several cell cycle related genesets35 for all cancer clusters. The y-axis depicts the GSVA enrichment score, individual clusters and patients are indicated on the x-axis. Each box shows the median and the middle quartiles. Panel A shows the GSVA enrichment score of the M to G1 Phase geneset. Panel B shows the GSVA enrichment score of the S Phase geneset. Panel C shows the GSVA enrichment score of the G2 Phase geneset. Panel D shows the GSVA enrichment score of the G2 to M Phase geneset.

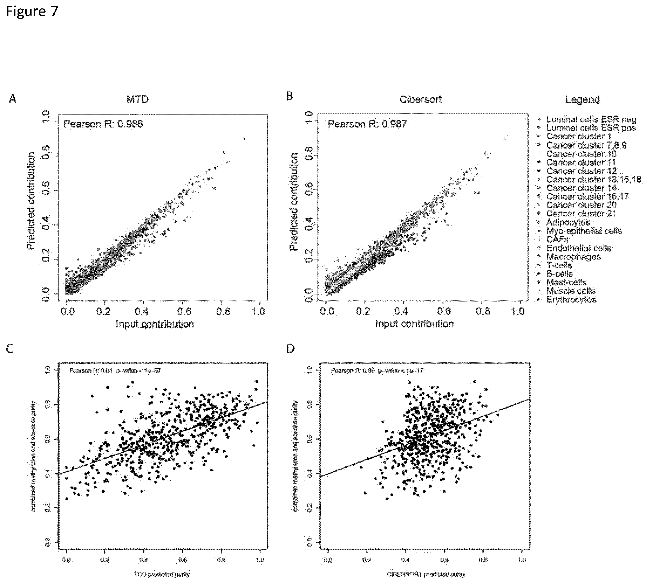

**Figure 7. TCD has a higher predictive value then CIBERSORT.**

Panel A and B show the in silico performance of TCD and CIBERSORT respectively. For each algorithm we constructed a training dataset with known input abundances for each cell type using 50% of the single-cell data. We then used the other 50% of the single-cell data to construct reference profiles for all of the cell types and used these as the reference profiles for TCD and CIBERSORT. For both plots the x-axis depicts the input fraction for each cell type. The y-axis depicts the predicted cell contribution by TCD and CIBERSORT for Panels A and B respectively. Panel C shows a dot plot between tumor purity of breast cancer samples from TCGA as predicted by TCD on the x-axis and the average tumor purity based on methylation and Whole Exome Sequencing (WES) on the y-axis. There is a high correlation (Pearson R 0.61) between tumor purity as predicted by TCD and the average tumor purity predicted from WES and methylation arrays. Panel D shows the same as Panel B but now for CIBERSORT. The correlation between tumor purity as predicted by CIBERSORT and WES and methylation array derived tumor purities is much lower (Pearson R 0.36).

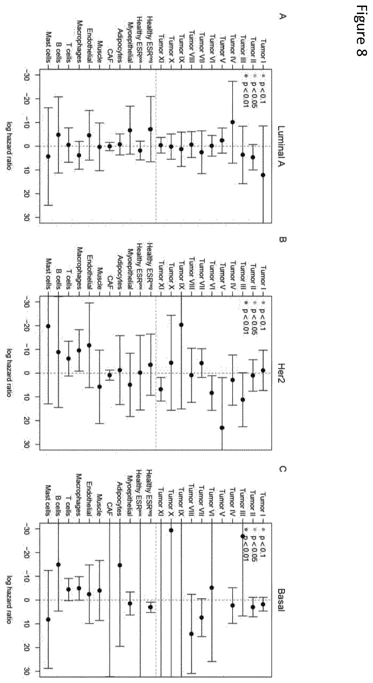

**Figure 8. Cox proportional hazard regression models for Luminal A, Her2 and Basal patients.**

This figure shows the Cox proportional hazard model for recurrence free survival for each of the cell types used in TCD for patients with Luminal A, Her2 and Basal tumors (Panel A, B and C respectively). Significance is indicated with black (not significant), green (fdr corrected p-value < 0.1), orange (fdr corrected p-value < 0.05) or red (fdr corrected p-value < 0.01).

Detailed description of the invention

The present invention aims to provide a method that allows for determining the cellular composition from a tumor sample by bulk RNA sequencing. Therefore, the present invention, in a first aspect provides a method for determining the cellular composition of a tumor from a subject suffering from cancer, the method comprising the steps of:

- a) performing bulk mRNA sequencing on tumor material obtained from the subject in order to obtain bulk sequencing data,
- b) comparing the bulk mRNA sequencing data of step a) with a reference library comprising clustered sequencing data in order to determine the relative contribution of each cluster comprised in the clustered sequencing data to the bulk sequencing data obtained from the tumor material obtained from the subject, and
- c) determining the cellular composition of the tumor material obtained from the subject based on the relative contribution of each cluster comprised in the clustered sequencing data to the bulk sequencing data.

In the method bulk mRNA sequencing is performed on tumor material from a patient. The tumor material may have been obtained by several means. For example, a tumor biopsy is or has been taken from a subject, and bulk mRNA sequencing is performed on this biopsy. This bulk mRNA sequencing data can be compared with a reference library comprising sequencing data from, for example, reference patients. The sequencing data of the reference library is clustered to reduce the amount of datasets that need to be compared. By making a comparison between the clustered sequencing data in the reference library and the bulk mRNA sequencing data obtained from the subject, the cellular composition of the tumor of the subject can be determined based on bulk mRNA sequencing data. This can be done by determining the optimal combination of the relative contribution of each cluster that would generate the bulk mRNA sequencing data obtained from the subject.

In the context of the invention, subject suffering from cancer or subject refers to a subject suffering from cancer for which subject the cellular composition of the tumor is to be determined. It is understood that the subject may be a human or an animal, such as for example a non-human mammal. The subject is preferably human.

Tumors generally comprise complex mixtures of tumorigenic cells (also referred herein as cancer cells) with their microenvironment which further comprises non-tumorigenic cells (also referred to herein as non-tumor cell types or non-tumorigenic cell types or non-cancer cells). Therefore, when used herein the term tumor refers to the totality of cells found within the tumor and/or tumor microenvironment, meaning both the tumorigenic cells and the non-tumorigenic cells interspersed between or located closely to the tumorigenic cells. It is well known that the presence or absence, or relative contribution of certain (tumorigenic or non-tumorigenic) cell types are of importance for, for example, survival rates or response to a specific therapy. Furthermore, tumorigenic cells can by subdivided in distinct sub-types. It is well known that tumors may comprise different subtypes, for example a tumor may comprise a subset of tumorigenic cells, i.e. one or more sub-types (or sub-clones), which do not respond to a certain therapy, whereas other sub-types present in the same tumor may respond to such treatment. In such case the used therapy may cause the tumor to shrink dramatically as the majority of cells responds to the tumor, however the patient will still relapse because of the non-responsive sub-types in the tumor. Further, certain non-tumorigenic cell types present in the tumor may either function to support or suppress tumor growth or survival. Therefore determining the cellular composition of the tumor is of importance, and could be instrumental in e.g. choice of treatment strategy.

In the context of the invention, cellular composition refers to the composition of the tumor or the tumor including the tumor microenvironment, meaning the totality of different tumorigenic cell sub-types and non-tumorigenic cells present in or in close proximity of the tumor, and which would normally be included in a biopsy of a tumor. The non-tumorigenic cells may be, but are not limited to, stromal cells and immune cells. It is further understood that the non-tumor cells may have a tumor supporting or a tumor suppressing role. Therefore, determining the cellular composition when used herein should be interpreted as determining which tumorigenic cell sub-types and which non-tumorigenic cell types are present in the tumor and/or the tumor microenvironment. When used herein, tumor or tumor material refers to the total of cellular components of different tumorigenic cell sub-types and non-tumorigenic cell types, its microenvironment, including the extracellular components.

The skilled person is aware that non-tumorigenic cells residing in the tumor environment may play an important role in tumor biology, for example either by supporting the tumorigenic cells or by suppressing or killing the tumorigenic cells. Typically stromal and immune cells can be found within the tumor, however the skilled person is aware that any type of cells may potentially play a role in tumor biology and therefore the term non-tumorigenic cells should not be interpreted as limited to (a) specific cell type(s).

In the context of the invention, tumorigenic cell sub-types should be interpreted as tumorigenic cells which are distinct from other tumorigenic cells found within the same tumor as determined by, for example, mRNA expression levels. Tumorigenic sub-types may also be referred to in the art as clones, tumor clones or sub-clones of tumorigenic cells within the same tumor. For example, a tumorigenic cell sub-type may refer to a sub population of tumorigenic cells within the same tumor with a different response to a drug when compared to the bulk of the tumorigenic cells found within the tumor, or when compared to other tumorigenic cell sub-types found within the tumor.

In the context of the invention, bulk mRNA sequencing should be interpreted as determining the expression levels based on the mRNA isolated from the sample, or the total mRNA in the sample (within the context of the invention the sample comprising tumor material obtained from the subject). RNA sequencing (also known as RNA-seq, also called whole transcriptome shotgun sequencing, WTSS), typically uses next-generation sequencing (NGS) to reveal the presence and quantity of mRNA in a biological sample at a given moment. Contrary to single-cell mRNA sequencing, bulk mRNA sequencing refers to sequencing performed on a sample consisting of a plurality of cells. For example such sample may be a tumor biopsy. The skilled person is aware that besides next generation sequencing, other methods can be used to determine the mRNA expression levels. For example, an alternative method is the use of microarrays. Therefore bulk mRNA sequencing, where used herein, should not be construed as limited to a certain method and encompasses any suitable method for determining mRNA expression levels of a sample, particularly a sample with a plurality of cells (Hrdlickova R. et al., 2017, Chu et al., 2012 and Wang et al., 2009).

The tumor material obtained from the subject may for example refer to a biopsy or a surgically removed piece of tumor tissue, but should be interpreted to include any means of directly or indirectly (e.g. through tissue culture) obtaining tumor material of a subject. Preferably the tumor material comprises both tumorigenic and non-tumorigenic cells, preferably the non-tumorigenic cells being cells from the tumor or tumor microenvironment.

The tumor microenvironment is the cellular environment in which the tumor exists, including but not limited to surrounding blood vessels, immune cells, fibroblasts, bone marrow-derived inflammatory cells, lymphocytes, signaling molecules and the extracellular matrix. The tumor and the surrounding microenvironment are closely related and interact constantly. Tumors can influence the microenvironment by releasing extracellular signals, promoting tumor angiogenesis and inducing peripheral immune tolerance, while the immune cells in the microenvironment can affect the growth and evolution of cancerous cells (tumorigenic cells). The skilled person understands how to obtain the tumor material for use in the methods of the invention.

The reference library according to the invention comprises clustered sequencing data. For example, a reference library may contain single-cell mRNA sequencing data obtained from one or more tumor samples. If cells from more than one tumor samples are obtained, this may refer to different tumors obtained from the same patient, or different tumors obtained from different patients. Preferably when obtaining more than one tumor sample the samples are obtained from tumors of the same type. In the library, this sequencing data is preferably clustered based on similarity in expression levels to form clusters of sequencing data obtained from each cell. Preferably, due to the clustering of single cell mRNA sequencing data based on expression levels, the clusters contain single-cell sequencing data obtained from cells with similar gene expression profiles. The clusters therefore preferably correspond to specific cell types or tumorigenic cell sub-types. For example single-cell mRNA sequencing data is obtained from 10 different cells within a tumor sample obtained from a patient. Clustering based on expression levels is performed on the single-cell mRNA sequencing data so that three clusters, "A", "B" and "C" are formed consisting of the single-cell mRNA sequencing data of 5, 3 and 2 cells respectively.

Preferably the reference library used in the method of the current invention is chosen such that, in case more than one tumor samples are used, the reference library comprises sequencing data obtained from tumor samples obtained from the same species as the subject. For example the samples are all obtained from humans. The sequencing data in the reference library may be obtained from humans or animals, such as non-human mammals, preferably the sequencing data in the reference library is from humans. In other preferred embodiments the sequencing data is obtained from the same type of cancer, for example all samples are obtained from breast cancer samples obtained from patients. In another preferred embodiment the samples are obtained from patients with the same sex, for example all samples are obtained from female patients.

In the context of the invention comparing the bulk mRNA sequencing data from the subject with the reference library should be interpreted as using the reference library to determine the relative contribution of each cluster of clustered single-cell mRNA sequencing data therein that would lead to the bulk mRNA sequencing data obtained from the subject. For example, the reference library contains a number of clusters corresponding to specific cell types and specific tumorigenic cell sub-types, each cluster having a specific expression profile, as obtained by single-cell mRNA sequencing from one or more patients. A computer algorithm may be used to calculate the optimal combination of each cluster, and their relative abundance, to create a virtual "bulk expression profile", which is identical or very similar to the bulk mRNA sequencing data obtained from the subject in the method of the invention. For example, the reference library contains clusters "A", "B" and "C", each corresponding to specific expression level profiles corresponding to the cell types identified by single cell mRNA sequencing in the patient. Bulk mRNA sequencing is performed on the tumor sample from the subject. By comparing it is determined that the bulk mRNA sequencing data corresponds to 3x cluster "A", 2x cluster "B" and 1x cluster "C". Doing so the cellular composition of the subject sample is determined, namely cells corresponding to expression profiles "A", "B" and "C" are present, further their relative abundances are determined, for example, cells corresponding to expression profile "A" are 3x as abundant as cell corresponding to expression profile "C".

In this context, determining the cellular composition of the tumor material obtained from the subject should be interpreted as providing a most likely combination of cells which would, based on the reference library, result in sequencing data which is comparable, preferably very similar, more preferably identical to the bulk mRNA sequencing data obtained in step a) of the method of the invention. The similarity between the combination of clusters and the bulk mRNA sequencing data can be determined by calculating the Pearson or Spearman correlation, or the Euclidean or Manhattan distance between the combination of clusters and the bulk mRNA sequencing data from the subject. This is not limited to these algorithms, but also other metrics of distance can be used to calculate this similarity. For example, if a computer algorithm is used to calculate the optimal combination of each cluster, a probability may be assigned to each possible combination, wherein the most probable combination of clusters has the highest likelihood of representing the actual cellular composition of the tumor material obtained from the subject. For example, the reference library consists of two clusters, "a" and "b". To construct a bulk mRNA sequencing data set highly similar as the one obtained from the subject, either a combination of 10x "a" and 1x "b" can be made or a combination of 5x "a" and 3x "b". As the latter option is more similar to the bulk mRNA sequencing data it is given a higher probability and therefore is assumed to represent the true composition of the cellular composition of the tumor sample obtained from the subject.

By clustering the sequencing data from the patient(s) in the reference library, the amount of "groups" or clusters with which the bulk sequencing data from the subject is to be compared is reduced considerably. This has the advantage that it greatly improves the comparison, and the chances that meaningful results are obtained. For example, single-cell mRNA sequencing data is obtained from patients for the reference library resulting in a total of 1000 unique single-cell mRNA sequencing data sets. When attempting to calculate the individual contribution of each of these 1000 sequencing data sets of the reference library on the total mRNA data from the subject, there will most likely be many combinations of these 1000 sequencing data sets that have a high similarity to the bulk mRNA sequencing data. Deciding which combination is the most likely one will therefore be very difficult if not statistically impossible. However, by clustering the single-cell mRNA sequencing data sets, for example, to 10 clusters of highly similar (based on mRNA expression profile) sequencing data sets, the comparison now needs to be made between 10 clusters and the bulk mRNA sequencing data. Much more statistically significant results may be obtained when determining the relative contribution of each of these groups to the bulk mRNA sequencing data due to the much lower amount of clusters. Clustering is done based on the similarity between the data sets obtained from the single-cells. If two single-cell data sets are highly similar, they will belong to the same cluster, whilst if those single cell data sets are very different, they belong to different clusters.

In a preferred embodiment, the step of comparing the bulk mRNA sequencing data with the reference library comprises using a tumor deconvolution algorithm on the bulk sequencing data.

A deconvolution algorithm is a process used to reverse the effects of convolution on recorded data. Deconvolution algorithms are known in the fields of image and data processing. For example, in the invention, the bulk mRNA sequencing data represents the convoluted recorded data, as it is the combined sequencing data obtained from many different cells within the tumor. The deconvolution algorithm tries to reconstruct the individual signals, in this case the individual mRNA expression profiles obtained from each of the cell types in the tumor, in such a way that when combined, these individual cell type specific expression profiles would form the bulk mRNA sequencing data.

When used herein, tumor deconvolution algorithm refers to any algorithm suitable for the deconvolution of bulk mRNA sequencing data from clustered sequencing data from a reference library. The main advantage of using a tumor deconvolution algorithm for performing the comparison between the reference library (obtained by single cell mRNA sequencing) and the bulk mRNA sequencing data, is that much more complex data sets can be used, and therefore better results can be obtained.

The performance of the deconvolution algorithm is dependent on the reference library. For example, if the cell types that are present in the bulk mRNA sequencing data are not present in the used reference library, such cell types will not be identified as such using a deconvolution algorithm. Therefore optimal construction of the reference library is of key importance. In view of this, it is preferred to use a reference library constructed from the same (or very similar) tumor type(s) as the tumor type of the subject for which the cell composition is being determined, for example tumors originating from the same tissue. This increases the chance that all cell types present in the bulk mRNA sequencing data are also included in the reference library. This may result in an improved determination of the cellular composition of the tumor of the subject.

Deconvolution algorithms are known to the skilled person, and for example described in Newman et al. Non limiting examples of deconvolution algorithms suitable for use as a tumor deconvolution algorithm in the invention are: from the reference profiles we can create a virtual tumor t,

$$t = \sum_{i=1}^{N} c_i \cdot R_i$$

where $c_i$ is the contribution of reference profile $i$ and $R_i$ is the reference profile $i$. We then use differential evolution to find the optimal values for $c$ such that

$$\min_{c} \left\| \sum_{i=1}^{N} c_i \cdot R_i - T \right\| \; subject\; to\; c_i \in [0,1] \; and \; \sum_{i=1}^{N} c_i = 1$$

where $T$ is the expression profile of the tumor from the subject as obtained through bulk mRNA sequencing, for which we want to find the cell type contributions. In this way the algorithm finds the optimal combination of cell type specific reference profiles so that, when combined, the reconstructed tumor from these reference profiles has the lowest possible Manhattan distance to the tumor from the subject.

In a preferred embodiment, the tumor deconvolution algorithm uses the reference library, and wherein the algorithm is used to calculate the relative contribution of each cluster in the reference library to the obtained bulk sequencing data.

In the context of the invention, using the reference library should be interpreted as providing the reference library as the plurality of input signals which when combined provide the output signal, wherein the plurality of input signals is represented by the clusters of single-cell mRNA sequencing data in the reference library. The output signal is represented by the bulk mRNA sequencing data. The algorithm is used to calculate the individual contribution of each of the separate input signals, corresponding to the clusters of single cell sequencing data from the patient(s) in the reference library, to obtain the output signal, corresponding to the bulk mRNA sequencing data obtained from the patient.

For example, the reference library comprises clusters of single cell sequencing data sets of tumorigenic cell subtype a, tumorigenic cell subtype b, and non-tumorigenic cells c, d and e. Each of these clusters have specific mRNA expression profiles, representing the input signals for the deconvolution algorithm. The output signal is represented by the bulk mRNA sequencing data. The deconvolution algorithm now uses the input signals and output signal to calculate a most probable combination of input signals to determine the cellular composition of the bulk mRNA sequencing data (the output signal). For example, based on the output signal, the most likely composition is 10x cell type a, 1x cell type b, 5x cell type c, 0x cell type d and 20x cell type e, as such combination of cells would result in exactly (or a signal highly similar to) the signal of the bulk mRNA sequencing and it is the most likely combination that would lead to such signal.

In the context of the invention, relative contribution of the cluster refers to the relative abundance of the cluster contributing to the bulk mRNA sequencing data. For example in the above example it can be determined that cell types a, b, c and e are present in the sample, while the relative abundance refers to the fact that, in the most probable combination of these cell types, 10 times more cells of type "a" compared to cells of type "b" contribute to the output signal (the bulk mRNA sequencing data).

In a preferred embodiment, both the bulk sequencing data and the reference library tumor materials are obtained from a tumor originating from or isolated from the same or comparable tissue types.

In order to improve the results of comparing the bulk mRNA sequencing data with the reference library, it is preferred that both are originating from or isolated from the same or comparable tissue types. In such case, a separate reference library may be constructed for tumors originating from or isolated from distinct tissue types. For example, separate reference libraries are created for breast cancer, colon cancer and melanoma. It is contemplated that within the invention, when obtaining tumor material from the subject, the most optimal reference library is chosen based on the tissue type, origin or cell type of the tumor of the subject, and where possible a reference library is selected derived from one or more tumors having the same or similar tissue type, origin or cell type. When using more than one tumor, the tumors may be derived from a single patient or from multiple patients.

In this context originating from the same or comparable tissue types should be interpreted as the tumor material is derived from a tumor originating in the same or comparable tissue type, however the material provided may have been isolated from a metastasized tumor, in which case the actual isolated material may be located in a different tissue type but originate from the same or comparable tissue type.

For the purpose of the invention, similar tissue type should preferably be interpreted as originating from the same basic tissue types, being epithelial, connective, muscular, and nervous tissue. More preferably similar tissue type is to be interpreted as tissues having a similar general function or being located in the same organ.

In a preferred embodiment, both the bulk sequencing data and the reference library are obtained from the same tumor type, the tumor type preferably being selected from the group consisting of breast cancer, melanoma, glioblastoma, lung cancer and colorectal cancer.

The principle demonstrated herein may be applied to any type of tumor, therefore the invention is not to be limited to specific tumor types. Preferred tumor types that work especially well with the method of the invention are breast cancer, melanoma, glioblastoma, lung cancer and colorectal cancer.

In a preferred embodiment a prediction is made based on the cellular composition of tumor material obtained from the subject, preferably said prediction is based on a characteristic,

comprising comparing the cellular composition of the tumor from the subject with at least one cellular composition of a comparison patient group with a known characteristic,

and wherein said prediction is preferably selected from the group consisting of:

- predicting progression free survival,
- predicting a treatment strategy,
- predicting a response to a treatment,
- linking cellular composition of a tumor to a treatment response,
- predicting recurrence free survival,
- predicting probability of metastasis formation, and
- predicting overall survival,

and wherein said prediction is based on comparing the cellular composition of the subject with the at least one cellular composition of the comparison patient group with known characteristic.

Preferably, the method of the invention is used to make a prediction for the subject based on the cellular composition of the tumor sample obtained from the subject as determined according to the method as described herein above. A prediction in the context of the invention is a prediction with respect to the subject from which the tumor sample is obtained, based on the cellular composition.

In the method, the prediction is based on a characteristic, wherein the characteristic is a state or property linked to the cellular composition of a tumor. For example the prediction is based on the finding that cell type "a" cause resistance to treatment "x". The characteristic can be determined by analysis of one or more tumors from the comparison patient group. The cellular composition of the tumors obtained from the comparison patient group can be determined by single cell sequencing, or it can be based on bulk mRNA sequencing using the method described above.

For example, based on the cellular composition as acquired by the invention and the clinical follow up analysis in a group of comparison patients, a poor prognosis was correlated to the presence of cell type "b" in the cellular composition of the tumor of those reference patients. In this case the characteristic can be "poor prognosis" and the prediction for the subject can be "the subject having a poor prognosis" if cell type "b" is found to be present in the tumor material obtained from the subject. For example a good response to treatment "x" is correlated with a high relative abundance of cell type "c", for example in case the tumor comprises at least 10% of cell type "c".

It is understood that the characteristic can also refer to a single state or property resulting from multiple combined events. For example in case good therapeutic results can be obtained with medicine x if cell type b is absent and cell type c is present, there are multiple events linked to the single characteristic "good therapeutic results with medicine x".

It is further understood that more than one characteristic can be linked to the same reference library. For example, the reference library consists of clusters of single cell sequencing data corresponding to cell types "a", "b" and "c", and the presence of cell type "b" corresponds to the characteristic good response to treatment "x" while high relative abundance of cell type "c" corresponds to the characteristic poor survival prognosis. Thus multiple predictions (in this case good response to treatment "x" and poor survival prognosis) for the subject may be made based and the multiple characteristics associated with the reference library.

In the context of the invention, comparing the cellular composition of the tumor from the subject with at least one cellular composition of a comparison patient group with a known characteristic should be interpreted as determining whether a similarity or difference exists in the cellular composition. Preferably the cell type for which a similarity or difference is found is linked to or associated with a characteristic as described above.

For the purpose of the invention, comparison patient group or patient group, is a group of individual patients suffering from cancer, from which tumor material has been obtained and for said tumor material a cellular composition has been determined, for example by single-cell mRNA sequencing or by bulk mRNA sequencing combined with the method described above. Said cellular compositions of the reference patients are used to determine a characteristic linked to the cellular composition. For example for a group patients, the cellular composition of the tumor was determined with either single-cell mRNA sequencing or by bulk mRNA sequencing combined with the method described above and it was found that patients with a certain cell type or relative abundance of a certain cell type, or with a combination of certain cell types, are more likely to later develop metastases. Thus the characteristic of developing metastases can by linked to the cellular composition of the patient group, namely the presence or absence of the specific cell type, or the relative abundance of a certain cell type, or a combination of certain cell types. The comparison patient group may also include (samples from) healthy people or non-tumor samples from persons suffering from cancer for purposes of normalizing or comparing the data. Bulk mRNA sequencing is then performed on the subject to determine the cellular composition using the method described above, so that the presence of a cell type, or absence of a cell type, or combination of cell types or relative abundance of a cell type linked the characteristic, in this example, increased chance of developing metastases. Based on these findings a prediction can be made whether the subject has increased chance of developing metastases or not.

The comparison patient group may refer to humans or animal, such as for example non-human mammals. Preferably the comparison patient group is human. More preferably the comparison patient group is the same species as the subject and/or the reference patient(s).

In the context of the invention the following preferred predictions may be made, although it should be understood that the invention is not limited to these predictions: predicting progression free survival, predicting a treatment strategy, predicting a response to a treatment, linking cellular composition of a tumor to a treatment response, predicting recurrence free survival, predicting probability of metastasis formation, and predicting overall survival.

Progression-free survival (PFS) is the length of time during and after the treatment of a disease, such as cancer, that a patient lives with the disease but it does not get worse. For example it was found in group of comparison patients that the presence of cell type "d" in the tumor correlates to a PFS which is twice as long as for patients where cell type "d" is not present in the tumor. Based on the presence of cell type "d" in the tumor material of the subject a prediction of a PFS which is twice as long can be made.

A treatment strategy refers to the most promising treatment, which can, among others, be based on the highest chance of curing, longest survival, best recurrence free survival rate or best quality of life, or combinations of these. For example it was found in comparison patient group that patients with cell type "e" in the tumor respond to treatment "x" but not to treatment "y". Based on the presence of cell type "e" in the tumor material of the subject the prediction can be made that a treatment strategy is to treat with treatment "x".

A response to a treatment can be any response to a treatment or a therapy, such as, for example, reduction of tumor growth or reduction of tumor size. Linking cellular composition of a tumor to a treatment response refers to linking the presence or absence of at least cell type, or the relative abundance of at least one cell type in the tumor to a treatment response, wherein said linking may be referring to improved or worsened response when compared to when said cell type is not present or absent, or present in a different relative abundance. For example it was found in group of comparison patients that the presence of cell type "d" in the tumor correlates to a better response to treatment "x" as for patients where cell type "d" is not present in the tumor. Based on the presence of cell type "d" in the tumor material of the subject a prediction of a better response to treatment "x" can be made.

Recurrence free survival is also known as disease free survival or relapse free survival, and, in cancer, refers to the length of time after primary treatment for a cancer ends that the patient survives without any signs or symptoms of that cancer. For example it was found in group of comparison patients that the presence of cell type "d" in the tumor correlates to a recurrence free survival which is twice as long as for patients where cell type "d" is not present in the tumor. Based on the presence of cell type "d" in the tumor material of the subject a prediction of a recurrence free survival which is twice as long can be made.

Probability of metastasis formation is the chance that development of secondary malignant growths at a distance from a primary site of cancer occurs.

Overall survival refers to the length of time from either the date of diagnosis or the start of treatment for a disease, such as cancer, that patients diagnosed with the disease are still alive. For example it was found in group of comparison patients that the presence of cell type "d" in the tumor correlates to an increased chance of metastasis formation when compared with for patients where cell type "d" is not present in the tumor. Based on the presence of cell type "d" in the tumor material of the subject a prediction of increased chance of metastasis formation can be made.

In the context of the invention, the prediction being based on comparing the cellular composition of the subject with the at least one cellular composition of the comparison patient group with known characteristic means that the prediction relates to the cellular composition and is based on a characteristic linked to the cellular compositions of the patient group. As explained above, in practice this means that a characteristic is determined in a comparison patient group, for example "increased chance of metastasis" which is based on the cellular composition, for example "presence of cell type "d"", the prediction "increased chance of metastasis" can be made for the subject based on the presence of cell type "d" in the tumor material obtained from the subject. In a preferred embodiment of the invention said characteristic is based on at least two comparison patient groups.

Preferably the prediction is based on two or more patient groups, and more preferably the characteristic is determined based on a difference in the two patient groups. For example one comparison patient group being treated with medicine x and the other comparison patient group being treated with medicine y. This allows to make a comparison between two comparison patient groups and link differences between these groups to a characteristic, allowing a prediction. For example, the presence of cell type "e" leads to good results for treatment with medicine x but not for treatment with medicine y. In such case a prediction can be made for a subject in case cell type "e" is found to be present that the subject will likely have a good response to treatment with medicine x but not to treatment with medicine y.

In a preferred embodiment the reference library used in step b) is a library that was, independently, obtained by a method comprising the following steps:

- i) performing single-cell mRNA sequencing on individual cells isolated from tumor material obtained from at least one reference patient in order to obtain a sequencing data set for each sequenced cell,
- ii) generating a clustering of the sequenced single cells based on the sequencing data sets of step i) in order to obtain a reference library comprising clustered sequencing data sets.

One of the main advantages of the method of the invention is that once a reference library has been constructed, the prediction can be made based on bulk sequencing data and there is no need to perform single-cell mRNA sequencing on each subject. Therefore, the constructing of the reference library by single-cell mRNA sequencing should be seen as a separate method, meaning it is not part of the method of determining the cellular composition of a tumor obtained from a subject.

The reference library used in the method of the invention may be obtained or is obtainable by the method described herein. The reference library obtained by or obtainable by the method described herein is suitable for use in the above described method for determining the cellular composition of a tumor from a subject.

Single-cell mRNA sequencing, also referred herein as single cell sequencing is known in the art as a method to determine the mRNA expression levels on a cellular level, meaning the mRNA expression profile for each sequenced cell is determined individually. Single-cell sequencing techniques are known to the skilled person, and are for example described in Grun et al., 2015 and Grun et al., 2016. In short, cells are separated for example by FACSorting and RNA from single cells is extracted and sequenced. Sequence reads are mapped to the transcriptome in order to determine which mRNAs are expressed and the mRNA expression levels. A threshold may be set for minimal amount of mapped sequence reads and/or minimal amount of unique mRNA sequence reads for cells to be included.

In the context of the invention a reference patient is a person suffering from cancer from which person tumor material has been obtained and sequenced by single cell sequencing, in order to construct the reference library. Preferably one or more reference patients are used, more preferably more than one reference patient is used to construct the reference library, preferably 2, 3, 4, 5, 6, 7, 8, 9, 10 or more.

The term "sequencing data set" in the context of the method for obtaining the reference library refers to the sequencing data obtained from a single cell, preferably mRNA sequencing data. Therefore, the reference library comprises multiple sequencing data sets.

In the context of the invention clustering is the combining of sequencing data sets, preferably the sequencing data of individual cells, based on similarity of the mRNA expression pattern. It is known to the skilled person that there are many ways to perform clustering of single-cell data sets (see for example Grun et al., 2015 and Grun et al., 2016). Preferably the generated clusters comprise sequencing data sets obtained from similar or identical cell types, the cell types either being non-tumorigenic cell types or tumorigenic sub-types.

As used herein, mRNA expression pattern or mRNA expression levels refer to total of mRNA detected in an individual cell as determined by single cell sequencing.

In a preferred embodiment the reference library used in step b) is a library that was, independently, obtained by performing an additional step after step i) or after step ii), the step comprising:

- a) determining for each sequenced cell of step i) or each clustered data set of step ii) whether it is tumorigenic or non-tumorigenic.

Although not essential for constructing the reference library, it may be advantageous to determine which cells or clusters of sequencing data derived thereof are from tumorigenic cells and which are from non-tumorigenic cells. This can either be determined using the single cell sequencing data, or the clustered sequencing data. Therefore, this step can be performed after step i) or after step ii) of the method of constructing the reference library. The skilled person will be aware of several methods that can be used to determine whether the cells are tumorigenic or non-tumorigenic, the invention is not limited to any specific methods (Tirosh 2016, Tirosh 2016a). Non-limiting examples of determining whether the cells or clusters are tumor or non-tumor cells or clusters are:

- Comparing the data with data of known tumor/non-tumor origin. For example healthy tissue from a reference patient can be included as a reference to determine which single cell sequencing data or clusters are derived from non-tumor cell types. For example if the tumor material is from a breast tumor biopsy, a biopsy from the other (healthy) breast can be used as a reference.
- Comparing the expression profile of the single cell sequencing data or the clustered single cell sequencing data with known markers of non-tumorigenic cell types, or markers of known tumors.
- Determining the inferred copy number variance, as described herein.

In a preferred embodiment the reference library used in step b) is a library that was, independently, obtained by single cell mRNA sequencing of at least 10 cells, preferably at least 100 cells, more preferably at least 1000 cells, most preferably at least 10000 cells, obtained from tumor material obtained from at least one reference patient. Preferably the reference library was obtained by single cell mRNA sequencing of at least 10 cells, preferably at least 100 cells, more preferably at least 1000 cells, most preferably at least 10000 cells, obtained from tumor material obtained from each reference patient individually.

Preferably, when performing the single cell sequencing the data is curated by discarding sequencing data obtained for cells which generate too few sequencing reads, or where the diversity of unique RNA constructs identified is too low. Preferably cells generating fewer than 500, preferably 1000, more preferably 2000, even more preferably 5000 even more preferably 10000 most preferably 25000 mapped sequencing reads are discarded, and/or preferably cells in which fewer than 200, preferably 500, more preferably 1000, even more preferably 2000, most preferably 5000 unique transcripts are detected are discarded. Therefore, preferably the initial amount of cells used for single cell sequencing is preferably 1000 cells, more preferably 2000, even more preferably 5000 most preferably at least 10000.

In a preferred embodiment the reference library used in step b) is a library that was, independently, obtained by performing single-cell mRNA sequencing on a plurality of tumor materials obtained from a plurality of reference patients and/or a plurality of tumor materials obtained from the same reference patient in step i).

In a preferred embodiment the reference library is obtained by performing single cell mRNA sequencing on tumor materials obtained from 2, preferably 3, 4, 5, 6, 7, 8, 9, or 10, or more than 10 reference patients, and/or obtained from 2, preferably 3, 4, 5, 6, 7, 8, 9, or 10, or more than 10 tumor materials obtained from the same reference patient in step i).

In a preferred embodiment the reference library used in step b) is a library that was, independently, obtained by a single-cell mRNA sequencing clustering algorithm in step ii).

Single cell sequencing clustering algorithms are known in the art, therefore it is within the domain of the skilled person to cluster the single cell sequencing data to obtain clusters of sequencing data (Grun et al., 2015 and Grün et al., 2016). For example, RaceID may be used, therefore in a more preferred embodiment the reference library was obtained by RaceID clustering in step ii). A suitable non-limiting example is RaceID, wherein ward.D2 hierarchical clustering is used, therefore in a more preferred embodiment the reference library was obtained by RaceID, using ward.D2 hierarchical clustering in step ii). In a most preferred embodiment the reference library was obtained by RaceID, wherein ward.D2 hierarchical clustering on the Manhattan distances is used in step ii). However, the skilled person will be aware that many other suitable clustering algorithms can be used, therefore the invention is not limited to any particular clustering algorithm described or used herein. Some examples of single-cell mRNA sequencing clustering algorithm are Seurat, Monocle and Backspin (Satija R. et al. 2015, Qiu et al. 2017, Zeisel et al. 2015).

Because the clustering is based on similarity in mRNA expression levels (mRNA expression profiles), the clusters tends to contain single cell sequencing data sets derived from similar or identical cell types. Based on marker genes expressed within the clusters or single cell sequencing data sets the cell types may be determined.

In a preferred embodiment the reference library used in step b) is a library that was, independently, obtained by the clustering of step ii) being followed by an additional step of:

- iii) assigning to each cluster of sequencing data in the reference library a cell type or tumorigenic cell sub-type based on mRNA expression levels.

Based on the expression of markers genes, for each cluster of single sequencing data it can be determined what the cell type is in case it relates to a non-tumorigenic cluster or what the sub-type is in case it relates to a tumor cluster. This additional step is particularly useful so an additional round of grouping can be performed, further reducing the amount of groups (grouped clusters) to improve the quality and statistical significance of the comparison of the bulk mRNA sequencing data with the reference library. For example the following mRNA transcripts were identified using single cell sequencing on tumor material obtained from a reference patient as follows: cell "x" mRNA-1 12 reads, mRNA-2 0 reads and mRNA-3 3 reads, cell "y" mRNA-1 10 reads, mRNA-2 0 reads and mRNA-3 4 reads, and cell "z" mRNA-1 5 reads, mRNA-2 10 reads and mRNA-3 3 reads. Based on similarity in mRNA expression profile, cells "x" and "y" are clustered together in cluster "a", while cell "z" differs from the previous cells and is clustered in a separate cluster "b".

Therefore in an even more preferred embodiment, in a preferred embodiment the reference library used in step b) is a library that was, independently, obtained by performing an additional step of:

- iv) grouping the clustered sequencing data based on the assigned cell types and tumorigenic cell sub-types, and wherein all clustered sequencing data that were assigned the same cell type or same tumorigenic cell sub-type are grouped to form a group of clusters.

Preferably the step is performed after step iii). The further reduction through grouping of the number of clusters in the reference library will generate more meaningful results when the bulk mRNA sequencing data is compared with the reference library, particularly when a deconvolution algorithm is used.

In a preferred embodiment the reference library used in step b) is a library that was, independently, obtained, and wherein the tumors obtained from the reference patients are tumors originating from the same or comparable tissue types.

In case tumors from more than one reference patients are used to obtain the reference library, it is preferred that the tumors originate from the same or comparable tissues. Although a single all-purpose reference library is contemplated within the context of the invention, more accurate results may be obtained by generating separate reference libraries for tumors originating from the same or comparable tissue types. Therefore, preferably the reference library is constructed from multiple tumors obtained from one or more reference patients that originate from the same or comparable tissue types. More preferably, the reference library is constructed with tumor materials from one or more reference patients wherein the tumor type is the same. Therefore, in a more preferred embodiment the reference library used in step b) is a library that was, independently, obtained from the same tumor type, preferably wherein the tumor type is selected from the group consisting of breast cancer, melanoma, glioblastoma, lung cancer and colorectal cancer.

It is further envisioned as part of the invention that method of making a prediction as described above is not limited to the method of described herein to determine the cellular composition. The prediction can be performed for any subject suffering from cancer, for which the cellular composition of the tumor is known. Therefore in a further aspect the invention relates to a method for making a prediction for a subject suffering from cancer, wherein the subject has a tumor with known cellular composition and wherein a prediction is made based on the cellular composition of tumor from the subject, preferably said prediction is based on a characteristic,

comprising comparing the cellular composition of the tumor from the subject with at least one cellular composition of a comparison patient group with a known characteristic,

and wherein said prediction is preferably selected from the group consisting of:

- predicting progression free survival,
- predicting a treatment strategy,
- predicting a response to a treatment,
- linking cellular composition of a tumor to a treatment response,
- predicting recurrence free survival,
- predicting probability of metastasis formation, and
- predicting overall survival,

and wherein said prediction is based on comparing the cellular composition of the subject with the at least one cellular composition of the comparison patient group with known characteristic.

In an aspect the invention relates to a method for preparing a reference library, the method comprising the following steps:

- i) performing single cell mRNA sequencing on individual cells isolated from tumor material obtained from at least one reference patient in order to obtain a sequencing data set for each sequenced cell,
- ii) generating a clustering of the sequenced single cells based on the sequencing data sets of step i) in order to obtain a reference library comprising clustered sequencing data sets.

Besides a method for determining the cellular composition of a tumor from a subject based on bulk mRNA sequencing, the invention further relates to a method for preparing a reference library. The reference library prepared by this method are particularly useful in the method for determining the cellular composition of a tumor from a subject according to the invention. The skilled person is aware how to perform bulk mRNA sequencing and how to perform clustering to obtain a reference library, as described above.

In a preferred embodiment step i) or step ii) is followed by an additional step of:

- a) determining for each sequenced cell of step i) or each clustered data set of step ii) whether it is tumorigenic or non-tumorigenic.

In a preferred embodiment said single cell mRNA sequencing is performed on at least 10 cells, preferably at least 100 cells, more preferably at least 1000 cells, most preferably at least 10000 cells obtained from at least one reference patient.

In a preferred embodiment said single cell mRNA sequencing in step i) is performed on a plurality of tumor materials obtained from a plurality of reference patients and/or obtained from a plurality of tumor materials obtained from the same patient.

In a preferred embodiment said clustering in step ii) is a single-cell mRNA sequencing clustering algorithm.

In a preferred embodiment the clustering is followed by an additional step of:

- iii) assigning to each cluster of sequencing data in the reference library a cell type or tumorigenic cell sub-type based on mRNA expression levels.

In a preferred embodiment step iii) is followed by an additional step of:

- iv) grouping the clustered sequence data based on the assigned cell types and tumorigenic cell sub-type, and wherein all clustered sequencing data that were assigned the same cell type or same tumorigenic cell sub-type are grouped to form a group of clusters.

In a preferred embodiment the tumors obtained from the reference patent are tumors originating from the same or comparable tissue types.

In a preferred embodiment each of the tumor materials are obtained from the same tumor type, wherein the tumor type is selected from the group consisting of breast cancer, melanoma, glioblastoma, lung cancer and colorectal cancer.

In an aspect the invention relates to a reference library obtainable or obtained by any of the methods according to the invention.

Example 1

*Methods*

**Flow cytometry on human breast tumor tissue**

Female breast cancer patients diagnosed with invasive carcinoma of no special type (previously known as invasive ductal carcinoma, 9 patients) or Ductal Carcinoma In Situ (DCIS) (1 patient) who underwent mastectomy at the Netherlands Cancer Institute were included in the study (n=10). Following pathological examination, tumor material was kept on ice in DMEM/F12 + GlutaMAX (GIBCO, Invitrogen Life Technologies), 1% HEPES, 1% penicillin-streptomycin from that point onwards. Tumors were mechanically dissociated using sterile scalpels and enzymatically digested with 25 $\mu$g/ml DNase I (Roche) and 15 Wunsch units TH Liberase /ml (Roche) diluted in PBS at 37 °C for 35 minutes, followed by mashing through a 70 $\mu$m filter (BD Falcon) while adding DMEM/F12 + GlutaMAX (GIBCO, Invitrogen Life Technologies), 1% Hepes, 1% Penicillin-Streptavidin. Tumor cells were blocked in 80% FACS buffer (5 mM EDTA in PBS supplemented with 5% fetal calf serum / 20% serum mix (50/50 normal goat serum (monx10961, Monosan) and Fc$\gamma$II/III receptor blocking serum 2.4G2 (kind gift from L. Boon, Bioceros, The Netherlands)) for 10 minutes on ice before labeling with EpCAM PE (1B7, eBioscience), CD14-FITC (M$\phi$P-9, bdBiosciences) labeled antibodies for 40 minutes on ice. DAPI was added as a cell death marker, and cells were sorted either on a FACS Ariall Special Ordered Research Product (BD Biosciences), on a FACS Jazz (BD biosciences), or on a FACS Fusion (BD biosciences). A broad FSC SSC gate was followed by a gate excluding doublets, after which the DAPI negative cells were selected. These DAPI negative cells were sorted into 384-well plates and further processed for single cell sequencing. To obtain enough cancer cells and macrophages, additionally EpCAM positive and CD14 positive cells were selected and index-sorted into 384-well plates and further processed for single cell sequencing. For the first 3 out of 10 patients, only EpCAM positive and CD14 positive cells were isolated and sequenced.

**Immunohistochemistry**

Immunohistochemistry of samples was performed on a BenchMark Ultra autostainer (Ventana Medical Systems). Briefly, paraffin sections were cut at 3 $\mu$m, heated at 75°C for 28 minutes and deparaffinized with EZ prep solution (Ventana Medical Systems). Heat-induced antigen retrieval was carried out using Cell Conditioning 1 (CC1, Ventana Medical Systems) for 32 minutes at 95°C, for Anti-Human Epithelial Antigen this retrieval was followed with a protease 3 (Ventana Medical Systems) treatment for 4 minutes. E-Cadherin was detected using clone NCH-38 (1/200 dilution, 32 minutes 37°C, DAKO) and the Anti-Human Epithelial Antigen using clone Ber-EP4 (1/100 dilution, 32 minutes 37°C, DAKO). For E-Cadherin an additional amplification step (Optiview Amplification kit, Ventana Medical Systems) was selected in the protocol to amplify this primary antibody. Bound antibody was detected using the OptiView DAB Detection Kit (Ventana Medical Systems). Slides were counterstained with Hematoxylin and Bluing Reagent (Ventana Medical Systems). Percentages of stromal and cancer cells were manually scored by an independent pathologist who was blinded to the results of TCD.

**Single cell mRNA sequencing**

Single cell mRNA sequencing was performed using Sort-seq as described in Muraro *et al*. Single cell libraries were sequenced on Illumina NextSeq500 with 75bp paired end reads. Read1 contains the cell barcode and Unique Molecule Identifier, read2 was mapped to the hg19 RefSeq transcriptome using Burrows-Wheeler Aligner with standard parameters.

**mRNA isolation from FFPE material**

For 7 patients tissue was isolated from deparaffinised tissue sections and RNA was isolated using All prep DNA/RNA kit (Qiagen 80234). For two patients there was not enough material to extract RNA and for one patient the quality of RNA was not good enough to generate sequencable libraries. Concentrations of RNA were measured using NanoDrop. Quality and quantity of the total RNA was assessed by the 2100 Bioanalyzer using a Nano chip (Agilent, Santa Clara, CA). Total RNA samples having DV200>30% were subjected to library generation. Libraries were generated using TruSeq RNA Access Library prep-kit and sequenced with 65bp single reads on a HiSeq2500 using V4 chemistry (Illumina Inc., San Diego).

**Bulk mRNA sequencing analysis**

Bulk mRNA sequencing data was analysed using the RSEM package, reads were aligned to the hg38 reference genome using STAR with default parameters. Transcripts per million values were used in further analyses. Subtype classification of bulk samples was done using the PAM50 classifier from the genefu R package (https://www.pmgenomics.ca/bhklab/software/genefu)[19].

**Single cell clustering and cell type identification**

Single cell transcriptomes were filtered for cells that had at least 2,000 unique transcripts and subsequently normalized to 2,000 transcripts per cell. Single cells were clustered using RaceID3 with the following parameters, clusterFUN: hclust, metric: manhattan, linkage: ward.D2. Cell types were identified based on the expression of well-known cell specific marker genes; myo-epithelial cells (*KRT5, KRT14, KRT17*), muscle cells (*MYH11, MCAM, MYL9, ACTA2*), endothelial cells *(VWF, CDH5, KDR, PTPRB, PECAM1, CD34, SOX18)*, cancer-associated fibroblasts (*COL1A1, CXCL12, SPARC, VIM, POSTN, MMP11, PN1)*, adipocytes (*APOD, DLK1, SCARA5, CFD*), T-cells (*CFD, GZMK, CD3D, CD3E, CCL5, PTPRC, CD8A, CD7*), B-cells (*CD79A, IGJ, SSR4, IGLL5, IGLL1*), macrophages (*CD14, IL1B, CLEC5A, CD74, HLA-DPA1, CD68, CD163, CSF1R)*, mast cells (*CTSG, CPA3, CMA1*), erythrocytes (*HBA2, HBB*) and epithelial cells (*ESR1, AR, PGR, ERBB2, KRT15, KRT23, KRT81, CDH1, EPCAM, CD24, CD44, MKI67, MUC1*)

**Copy number variance estimation**

Copy number variance (CNV) estimation based on single-cell mRNA data was performed. Briefly, gene expression counts were log transformed and mean centred. Gene expression counts were subsequently averaged by taking a running mean with a 100-gene window along the genome. The average profile of the healthy cells from patient 7 was used to normalize the CNV profiles.

**Subtype classification**

Single-cell subtype classification was done using the Genefu R package (https://www.pmgenomics.ca/bhklab/software/genefu) with the PAM50 classifier. Genefu was used using default parameters. Probability scores for the 5 different subtypes were calculated.

**Gene Set Variation Analysis**

Gene Set Variation Analysis (GSVA) was done using the MSigDB C2 gene set collection and the GSVA R package. Following single-cell GSVA, cluster specific significant enrichment or depletion of each gene set was calculated using a Wilcoxon test comparing the geneset enrichment in the single cells from each cluster to all single cells not belonging to that cluster.

**Tumor Cell Deconvolution Algorithm**

The Tumor Cell Deconvolution (TCD) algorithm aims to perform deconvolution of bulk mRNA sequencing samples into contributions from individual cell types. For the non-cancer cell types, averaging the RaceID cluster that represents each individual cell type created the reference profile. For the non-cancer cell types that were represented by multiple RaceID clusters, all RaceID clusters belonging to that cell type were averaged. The reference profiles of the cancer cells were based on the RaceID clustering where non-cancer cell types were excluded. To reduce the number of cancer cell reference profiles we combined clusters with the most similar gene expression profiles, thereby creating 12 reference profiles for non-cancer cells and 11 reference profiles for cancer cells. Both the expression profiles from the real tumors and the reference profiles are normalized to $10^6$ counts prior to performing TCD. TCD works as follows: from the reference profiles we can create a virtual tumor *t,*

$$t = \sum_{i=1}^{N} c_i \cdot R_i$$

where $c_i$ is the contribution of reference profile *i* and $R_i$ is the reference profile *i.* We then use differential evolution to find the optimal values for *c* such that

$$\min_c \left\| \sum_{i=1}^{N} c_i \cdot R_i - T \right\| \; subject \; to \; c_i \in [0,1] \; and \; \sum_{i=1}^{N} c_i = 1$$

where $T$ is the expression profile of the real tumor for which we want to find the cell type contributions. In this way TCD finds the optimal combination of cell type specific reference profiles so that, when combined, the reconstructed tumor from these reference profiles has the lowest possible Manhattan distance to the real tumor. We use an R implementation of the Differential Evolution (5000 iterations with a population size of 20 and cross-over rate of 0.7) algorithm to find this optimal combination of the reference profiles. The TCD code and the reference profiles are included in this paper and can be used for the deconvolution of any set of breast cancer samples.

**Cell type contribution predictions using CIBERSORT**

CIBERSORT was used with default parameters using the single-cell derived reference profiles described above as reference profiles.

**Training data set**

We generated an *in silico* training set by randomly dividing the isolated cells from each patient into two sets. The first set was used to generate *in silico* tumors, thereby creating an *in silico* bulk mRNA sample. The other set was used to create the reference profiles for TCD as described above. Next TCD was run with the *in silico* tumors as target and the TCD predicted cell type contributions were compared to the known input contributions that were used to create the *in silico* tumors.

**TCD validation**

To investigate the performance of TCD on real patient data TCD and CIBERSORT were used to predict cell type contributions on breast cancer samples from The Cancer Genome Atlas (TCGA) for which mRNA sequencing data was available. The sum of the tumor cell type contributions, which serves as an estimate for tumor purity, was compared to the average tumor purity as predicted by ABSOLUTE or methylation arrays. For further validation TCD was used to predict cell type contributions on the bulk mRNA sequencing profiles from the 7 patients from which both single-cell and bulk mRNA sequencing data was available. These contributions were then compared to histology based tumor, stroma, lymphocyte and fat content from H&E stained tissue sections from each of these patients. For this, the summed cancer cluster contributions were compared to tumor content, summed T cell, B cell and macrophage contributions were compared to lymphocytes, stroma was directly compared to CAF contribution and fat was directly compared to adipocyte contribution. For the comparison between single-cell contributions to the cancer clusters and TCD contributions to the cancer clusters TCD was ran using all 23 cell types as reference profiles. Following TCD, the contribution of the cancer clusters was rescaled so that for each patient their total summed to 1. The fraction of single cells belonging to a cancer cluster was calculated by dividing the number of single cells belonging to a cancer cluster by the total number of cancer cells from that patient.

**Patient cohorts**

To validate the TCD, we have used the following patient cohorts: the Cancer Genome Atlas (TCGA) breast cancer cohort as available via https://cancergenome.nih.gov/cancersselected/breastductal and 664 patients from the randomized MATADOR breast cancer trial from which for 528 patients mRNA sequencing data was available and which has a median follow-up time of 7 years. In short for the MATADOR breast cancer cohort, patients were enrolled in the study between 2004 and 2012 and were randomized to six cycles of doxorubicin 60 mg/m² Cyclophosphamide 600 mg/m² every 2 weeks (ddAC) or six cycles docetaxel 75 mg/m² doxorubicin 50 mg/m² Cyclophosphamide 500 mg/m² every 3 weeks (TAC). Tumors range between pT1-T3 and pN0-3 and there were no significant differences in clinopathological characteristics. The primary objective of the MATADOR trial was to investigate whether a breast cancer derived gene expression profile could be developed to select the best chemotherapeutic regimen (ddAC or TAC) for an individual patient.. The analysis presented in this manuscript are exploratory subgroup analysis.

**Statistics survival curves**

To measure the influence of cell type contribution on recurrence free survival, the mean and standard deviation of the contribution of a cell type (e.g. macrophages) were determined across all patients in this group (e.g. luminal A tumors). Next, for each patient we determined whether the contribution of this particular cell type was low (< mean - 1 SD), medium (> mean - 1 SD and < mean + 1 SD), or high (> mean + 1 SD). The distribution of contributions for some cell types is asymmetric, therefore sometimes the mean - 1 SD < 0, in these cases we defined the low contribution category (no contribution) as < 0.01 . The Kaplan-Meier recurrence free survival probability curves were obtained using the kmTCGA R package with standard parameters. For the Kaplan-Meier curves p-values were obtained by performing a LogRank test. Cox proportional hazard regression model were calculated independently for each cell type and corrected for AJCC tumor stage. This resulted in 23 (number of reference cell types) Cox regression models for which the p-values for the influence on recurrence free survival were corrected for multiple testing using Benjamini-Hochberg false discovery rate (fdr) correction. The Cox proportional hazard regression model on treatment was calculated per cell type per treatment arm, again p-values were corrected for multiple testing using Benjamini-Hochberg fdr correction.

## Results

### Single cell mRNA sequencing of human breast cancer

From the freshly isolated breast tumors of ten patients, we isolated a set of randomly picked cells from the tumor (DAPI-negative) using flow cytometry (Fig 1A). To obtain enough cancer cells and macrophages, we additionally isolated respectively EpCAM-positive and CD14 positive cells (for the first three patients only Epcam-positive and CD14-positive cells were isolated). Similar to the incidence of breast cancer subtypes in the US, the majority of the analyzed tumors belong to the Estrogen Receptor (ER)/ Progesteron Receptor (PR) positive invasive carcinomas of no special type (previously known as invasive ductal carcinoma), and one to the triple-negative subtype (Fig 1B). The tumor size within the patients ranged from T1 to T3, two patients had clinically assessed lymph node metastases, and none of the patients had distant metastases at time of surgery. Half of the patients underwent neoadjuvant treatment with cytotoxic or endocrine therapy with a partial response. Next, we processed the samples for single-cell mRNA sequencing and characterized the expression profiles of 5,722 cells, including cancer and non-cancer cells.

### Identification of the various cell types present in the tumor microenvironment

To identify all cellular components of these breast tumors, we performed RaceID clustering of the entire population of cells from all patients combined and revealed 36 clusters of cells with distinct expression profiles (Fig 1C). Of these, multiple clusters contained cells from all patients (Fig 1C-D). The gene expression profiles showed that most of the clusters comprising of cells of multiple patients represent the various cell types present in the tumor microenvironment including cancer-associated fibroblasts (CAFs), myoepithelial cells, adipocytes, muscle cells, endothelial cells, macrophages, T cells, B cells, and mast cells (Fig 1E). Importantly, we did not observe any general differences in expression profiles between microenvironmental cells from neo-adjuvant treated and treatment-naive patients, suggesting that neo-adjuvant treatment did not have a major influence on the expression profile of these cells.

Next, to detect distinct sub-clones with subtle differences in expression profiles, we excluded all micro-environmental cell populations from our analysis. Moreover, we also included non-cancerous epithelial (i.e. EpCam positive) cells from the contralateral breast of patient 7, which did not contain a tumor. On all these cells combined, we again performed RaceID clustering (Fig 2A, B). This analysis revealed 21 clusters of cells with distinct expression profiles. Of these clusters, clusters 2, 3, 4, 5 and 6 contain cells from multiple patients, and can therefore potentially be a non-cancerous epithelial population of cells (Fig 2B). Indeed, the non-cancerous epithelial cells of patient 7 were present in clusters 2 to 6, suggesting that the cells in these clusters are non-transformed cells (Fig 5A). Next, we confirmed the non-transformed nature of these cells by analyzing the copy-number variation (CNV) patterns of these cells. As expected for non-transformed cells, cells from clusters 2 to 6 showed a lower variance of the CNV across the genome compared to the cells in the other clusters (Fig 5B-C). Moreover, the CNV variance in clusters 2 to 6 is comparable with the CNV variance in the non-cancerous cells from the contralateral breast from patient 7. Strikingly, cells in other clusters displayed extensive chromosomal copy number aberrations (Fig 5C-D), including gain of 16p and loss of 16q, which are typically found in luminal breast cancer, suggesting that these are clusters of cancer cells.

In contrast to previous efforts, the resolution of SORTseq enables us to identify distinct expression profiles of cancer cells within individual tumors (Fig 2B). The RaceID clustering showed that treatment status of the patient (neo-adjuvant-treated or treatment-naive) is not a major determinant of the clustering. Instead, cancer cells from most patients were distributed over multiple clusters and most clusters only contained cells from a single patient (Fig 2A-B). However, gene expression can fluctuate over time (e.g. during cell cycle) and variable expression levels of single genes may not always indicate distinct cell populations. Nevertheless, cell cycle related genes are not exclusively expressed by cells from just a few clusters, but instead are homogeneously distributed across cell clusters belonging to the same patient, indicating that cell cycle related genes are not a major determinant of the clustering of cancer cells (Fig 6). Combined this data suggest that breast tumors contain populations of cancer and non-cancer cells with distinct expression profiles.

**Single-cell analyses provide additional information on tumor heterogeneity complementary to classification into molecular subtypes**

To investigate whether the information on different expression profiles in distinct populations of tumor cells (i.e. different tumor clusters) is lost in bulk mRNA measurements, we analyzed the expression profiles of bulk and the corresponding single cell samples using the PAM50 classifier that classifies tumors into molecular subtypes: luminal A, luminal B, HER2-enriched, basal-like, and normal-like. Although the PAM50 is designed for treatment-naive tumors, we also analyzed the tumors that received neo-adjuvant treatment to test the agreement between single cell and bulk analysis. As expected from the highly heterogeneous composition of the tumors, with multiple cancer and non-cancer cell types, the classification of the tumors in one of the molecular subtypes is not a binary outcome. Although for most patients the tumors get the highest probability for belonging to the luminal A subtype, which is in accordance with the histological ER positive subtype (Fig 1B), the classifier also assigns a score for at least two other subtypes, though with lower probability (Fig 2C). In fact, when we classified each individual cancer cell, we found cancer cells from more than one subtype in every single tumor (Fig 2C). However, on average only 13 +/- 5 (mean +/- SD) genes of the PAM50 gene set were detected per single cell, leading to increased technical variation. It should be realized that the PAM50 and other classifiers are designed for bulk mRNA measurements and not to classify individual cancer cells into molecular subtypes. Nevertheless, this result urged the question whether this heterogeneity may contain important information on therapy response and survival prediction that are not taken into account with the currently bulk measurements. To investigate this, we analyzed the gene expression profiles of the clusters in more detail, and performed gene set variation analysis (GSVA). We found several clusters to be enriched for gene sets known to regulate cancer cell behavior, such as gene sets related to Myc signaling, TGFβ signaling, and Estrogen signaling (Fig 2D). Moreover, we found several gene sets to be differentially enriched between the closely related clusters 7, 8 and 9 and cluster 10, which all originate from patient 2 (Fig 2E). This data illustrates that a tumor can contain distinct populations of cancer cells with different expression profiles related to tumor progression and therapy effect, and that this information is lost upon analysis of bulk sequencing data by e.g. molecular classifiers such as the PAM50.

**Development of a computational algorithm to reveal tumor heterogeneity from bulk mRNA sequencing data**

The current clinically-used classifiers, such as PAM50, are based on bulk measurements that are dominated by the most abundant cell type in the tumor, whilst they will only be slightly influenced by the expression profiles of less abundant cell populations. Nevertheless, the abundance of minor cell populations can have a significant influence on therapy response and disease outcome [28]. In order to understand the contribution of all the different cell populations in a tumor and to relate these contributions to therapy benefit and disease outcome, it is relevant to extract the contribution of each cell population to the bulk tumor. Deconvolution algorithms aim to do exactly this, by disentangling the bulk signal into contributions from each of the individual components (Fig 3A). In order for this deconvolution to work, it is important to have well-defined gene expression profiles for each cell type present in the tumor, from here on referred to as reference profiles. CIBERSORT is a published deconvolution algorithm that uses reference profiles only from cells that are isolated from blood (i.e. immune cells). However, a recent study showed that the expression profile of immune cells isolated from the blood and tumors hardly overlap, which makes the deconvolution outcome less reliable. Therefore, we use reference profiles that are based on the expression profiles of cells isolated from tumors including the various populations of tumor cells that cannot be isolated from the blood (e.g. epithelial cells, endothelial cells, muscle cells and fibroblasts). For the non-cancer cell types, the reference profiles are based on the average expression profile of all cells within the RaceID clusters belonging to that cell type, as identified in Fig. 1C, D (Fig. 3B). The reference profiles of the epithelial cells (i.e. luminal and cancer cells) were based on the RaceID clusters in Fig. 2A, B (Fig. 3C). To increase the accuracy of deconvolution, we combined clusters with the most similar gene expression profiles, thereby creating 12 reference profiles for non-cancer cells and 11 reference profiles for cancer cells (indicated with roman numbers) (Fig. 3B and C).

To further increase reliability of deconvolution, we developed Tumor Cell Deconvolution (TCD). In contrast to CIBERSORT, that uses support vector machine regression, TCD uses a differential evolution algorithm to find the relative abundance of each cell type that, when combined into a virtual bulk sample, most closely reflects the gene expression profile of the original tumor (see methods for detailed description of TCD) (Fig 3A). TCD and CIBERSORT performed similarly on an *in silico* generated training data set (Fig 7A and B). To test the performance of both algorithms on real data, we first obtained the purity of breast tumors (i.e. contribution of cancer cells to the bulk of the tumor) from the breast cancer cohort of the TCGA. These studies determined the purity through Whole Exome Sequencing (WES) and methylation profiling. Next, we used TCD and CIBERSORT to deconvolve tumors from TCGA. To avoid potential biases based on different reference profiles, both algorithms were ran using the single-cell derived reference profiles described above. To calculate tumor purity all contributions from the cancer clusters were summed. Strikingly, the purity in the TCGA tumors found by TCD correlated better (R=0.61, P<$10^{-57}$) with the published purity numbers than the purity as determined by CIBERSORT (R=0.36, P<$10^{-17}$) (Fig 7C and D). To further validate TCD, we applied our algorithm on the bulk mRNA sequencing data from 7 patients in our dataset (for the other 3 patients, no mRNA sequencing data could be obtained) and observed a high correlation (R=0.89 p-value < $10^{-8}$) between TCD derived and independently scored histological cell type contributions (Fig 3D). Each non-cancer cell type has a specific expression profile that is distinct from all the other cell types (Fig 1E) and therefore the contribution of even small population of cells can be retrieved by deconvolution. Next, we questioned whether this also holds true for the contribution of the various cancer cell clusters with more subtle expression differences (Fig 2E). For this, we determined the relative contribution of each of the cancer cell clusters in every patient based on the single-cell sequencing data (Fig 3E) and compared this to the contribution of each cluster as predicted by TCD from the bulk sequencing data. For most of the tumors (6 out of 7), we were able to correctly establish the contribution of the main tumor cluster present in that patient (Fig 3E). From this, we conclude that TCD is able to successfully disentangle mRNA bulk sequencing data into contributions of all the different cell populations in a tumor and thereby reveals the cellular heterogeneity of a tumor.

**Identification of the cellular heterogeneity of tumors reveals new information on recurrence free survival and outcome upon differential treatment**

Next, we questioned whether identification of the cellular heterogeneity of tumors can reveal information on recurrence free survival and therapy response. To do this we applied TCD to the MATADOR trial: a randomized, controlled trial in which patients received adjuvant treatment with either dose-dense doxorubicin-cyclophosphamide (ddAC) or docetaxel-doxorubicin-cyclophosphamide (TAC) with a median follow-up of 7 years. Importantly, bulk mRNA sequencing data of the primary tumor was available for 528 patients that participated in the trial. From the mRNA profile of individual patients, we estimated the contribution of each of our reference cell types (Fig 3B and C) using TCD and tested whether identification of the cellular heterogeneity of the tumors could reveal disguised information on recurrence free and overall survival.

First, we tested whether the relative abundance of specific cell populations in the tumor, as determined by TCD, predicted differences in recurrence free survival of patients that received adjuvant treatment with either ddAC or TAC. Using the Cox proportional hazard regression model, we correlated the recurrence free survival to the contribution of each cell type for each PAM50 molecular subtype (Fig 4A and Fig 8). After false discovery rate correction of the p-values (Benjamini-Hochberg), we observed significant associations between the recurrence free survival and the contribution of tumor cluster III and VII, muscle, T cells and B cells for patients with Luminal B tumors. For the Luminal A, Basal and Her2 subtypes we did not find any significant associations of cell type contribution with recurrence free survival probability (Fig 8).

Next, we determined the mean and standard deviation of the contribution of cancer clusters III and VII, muscle, T cells and B cells across all patients with a luminal B tumor. Subsequently, for each patient we determined whether the contribution of this particular cell type was low (< mean - 1 SD), medium (> mean - 1 SD and < mean + 1 SD), or high (> mean + 1 SD). We found that high abundance of tumor cluster III and VII is associated with a significant reduction in recurrence free survival (Fig 4B and C). Interestingly, the expression profiles of these clusters are enriched for gene sets involved in cytoskeleton remodelling, TGFβ signaling and metastasis (Fig 2D). In addition to the cancer cells, we found that populations of non-cancer cells can influence recurrence free survival upon treatment. A high abundance of muscle cells (Fig 4D) also predicted a significant decrease in recurrence free survival. On the contrary, a high abundance of either T cells or B cells is indicative of increased recurrence free survival, suggesting an important role for the immune system in the recurrence free survival of Luminal B patients (Fig 4E and F).

Next, we tested whether the relative abundance of these various components can also have predictive power for the differential response to different therapies, i.e. in the MATADOR trial to ddAC or TAC. Recurrence free survival probabilities in each treatment arm were analyzed for all patients without separating tumors into different molecular subtypes. The Cox proportional hazard regression model (Benjamini-Hochberg false discovery rate corrected) showed that an increased contribution of $ESR^{neg}$ is indicative of an increased hazard ratio for patients that received TAC but not for patients that received ddAC (Fig 4G). Although there is no significant interaction between the $ESR^{neg}$ contribution and treatment in a Cox proportional hazard regression model, the Kaplan-Meier analyses show that high $ESR^{neg}$ contribution results in a significant reduction in the recurrence free survival probability for patients treated with TAC (p-value < 0.001) but not for patients treated with ddAC (p-value = 0.29) (Fig 4H). Moreover, the Cox proportional hazard model showed that increased contribution of either T cells or B cells is indicative of a decreased hazard ratio for patients that received TAC but not for patients that received ddAC (Fig 4G). However, only the interaction between the T cell contribution and treatment is signicifcant (p-value = 0.024). Indeed, the Kaplan-Meier analysis for T cells showed that patients with high T cell contribution have significantly better survival upon treatment with TAC than upon treatment with ddAC (Fig 4I). Combined, our data show that revealing the cellular tumor heterogeneity can uncover new information which has

EP 3 650 556 A1

predictive power for survival and treatment response and which can help to support future clinical decision making.

## Discussion

It is widely appreciated that cancer is a heterogeneous disease both in terms of cellular composition and therapy response. Prior efforts have tried to classify tumors based on histopathological and molecular features. Especially classification of tumors into intrinsic subtypes that also take genomic alterations into account, appeared to be a powerful tool to group patients with distinct outcomes and responses to therapies. In addition to the influence of cancer cells, it is increasingly appreciated that tumor-supporting stroma and immune cells may also influence therapy response. Therefore, we here dissected the cellular content of each tumor (i.e. cellular heterogeneity) and tested whether this information can be combined with current classification methods to guide treatment decisions. For example, we show that in the luminal B tumors, high abundance of muscle cells predicted decreased recurrence free survival upon therapy. Therefore, analyzing the cellular heterogeneity of a tumor, in addition to classifying tumors into molecular or intrinsic subtypes, may further help to guide treatment decisions.

Although single-cell mRNA sequencing is the most direct method to determine the cellular heterogeneity of a tumor, this technique is too labor intensive and expensive to be routinely performed for individual patients. TCD is an indirect method to obtain the same information, therefore it may serve as a fast and inexpensive alternative to single cell mRNA sequencing since it is based on often performed bulk mRNA sequencing from formalin fixed paraffin-embedded material. To obtain the most reliable predictions of tumor heterogeneity by TCD, good reference profiles of each cell type are required. A recent study showed that the expression profile of immune cells isolated from the blood and tumors hardly overlap, therefore we have constructed reference profiles from single cells isolated directly from breast tumors. We have identified 12 reference profiles of non-cancer cells, and all these profiles are based on multiple patients thereby eliminating patient-to-patient variation. Importantly, we observed that the expression profiles of these cells did not differ between neo-adjuvant treated and treatment-naive patients, suggesting that treatment does not have a major influence on the expression profiles of the non-cancerous cells. In addition to the reference profiles of non-cancerous cells, we have identified 11 reference profiles of cancer cells, most of which were unique for single patients. Therefore, the number of identified distinct cancer profiles may not be exhaustive. Nevertheless, many of the cancer clusters have upregulated genes involved in overlapping signaling pathways and processes, and therefore our identified profiles may have covered the most important biologically relevant tumor characteristics. For example, cancer clusters III and VII that had the largest influence on recurrence free survival in patients with luminal B subtype were significantly enriched for genes involved in TGFβ signaling and Epithelial to Mesenchymal Transition (EMT). TGFβ signaling and EMT are involved in therapy responses and metastasis and may therefore influence survival and therapy response regardless of the molecular subtype of the tumor. The idea that our cancer profiles determined in Luminal A tumors have covered the most important biologically relevant tumor characteristics in cancer populations is further supported by our observation that these profiles are able to predict recurrence free survival for an other subtype (i.e. Luminal B). Of all molecular tumor subtypes, Luminal A subtypes have the best prognosis, and therefore the dynamic range of cellular markers may be too small to detect differential survival.

Our data suggest that revealing the abundance of sub-populations of tumor cells by TCD can provide information on prognosis and can potentially even be used to predict response to therapy. For example, one difference between the TAC and ddAC regimen is the docetaxel treatment, and our data suggest that TCD (i.e. levels of ESR$^{neg}$ or T cells, see Fig 4H and I) can be used to predict clinical benefit of this additional taxane treatment. This illustrates that TCD and our reference profiles have the ability to reveal intra-tumor heterogeneity from bulk mRNA sequencing analysis in order to uncover additional information on recurrence free survival probabilities upon treatment. This approach therefore has the potential to further optimize and guide personalized medicine.

## References

Chu Y., RNA sequencing: platform selection, experimental design, and data interpretation. Nucleic Acid Ther. 2012 Aug;22(4):271-4. doi: 10.1089/nat.2012.0367. Epub 2012 Jul 25.

Grün D, Muraro MJ, Boisset JC, et al. De Novo Prediction of Stem Cell Identity using Single-Cell Transcriptome Data. Cell stem cell 2016;19:266-77.

Grün D, Lyubimova A, Kester L, et al. Single-cell messenger RNA sequencing reveals rare intestinal cell types. Nature 2015;525:251-5.Patel et al.

Hrdlickova R. et al., RNA-Seq methods for transcriptome analysis. Wiley Interdiscip Rev RNA. 2017 Jan;8(1). doi: 10.1002/wrna.1364. Epub 2016 May 19.

Kim C, Gao R, Sei E, et al. Chemoresistance Evolution in Triple-Negative Breast Cancer Delineated by Single-Cell Sequencing. Cell 2018.

Li B, Dewey CN. RSEM: accurate transcript quantification from RNA-Seq data with or without a reference genome. BMC Bioinformatics 2011;12:323.

Li H. et al., Reference component analysis of single-cell transcriptomes elucidates cellular heterogeneity in human colorectal tumors. Nat Genet. 2017 May;49(5):708-718. doi: 10.1038/ng.3818. Epub 2017 Mar 20.

Muraro M. et al., A Single-Cell Transcriptome Atlas of the Human Pancreas. Cell Syst. 2016 Oct 26;3(4):385-394.e3. doi: 10.1016/j.cels.2016.09.002. Epub 2016 Sep 29.

Tirosh I, Izar B, Prakadan SM, et al. Dissecting the multicellular ecosystem of metastatic melanoma by single-cell RNA-seq. Science 2016(a);352:189-96.

Tirosh I, Venteicher AS, Hebert C, et al. Single-cell RNA-seq supports a developmental hierarchy in human oligodendroglioma. Nature 2016(b);539:309-13. Patel AP, Tirosh I, Trombetta JJ, et al. Single-cell RNA-seq highlights intratumoral heterogeneity in primary glioblastoma. Science 2014;344:1396-401.

Qiu X. et al., Reversed graph embedding resolves complex single-cell trajectories. Nat Methods. 2017 Oct;14(10):979-982. doi: 10.1038/nmeth.4402. Epub 2017 Aug 21.

Satija R. et al., Spatial reconstruction of single-cell gene expression data. Nat Biotechnol. 2015 May;33(5):495-502. doi: 10.1038/nbt.3192. Epub 2015 Apr 13. Wang Z. et al., RNA-Seq: a revolutionary tool for transcriptomics. Nat Rev Genet. 2009 Jan;10(1):57-63. doi: 10.1038/nrg2484.

Zeisel A. et al., Brain structure. Cell types in the mouse cortex and hippocampus revealed by single-cell RNA-seq. Science. 2015 Mar 6;347(6226):1138-42. doi: 10.1126/science.aaa1934. Epub 2015 Feb 19.

CLAUSES

Clause 1. Method for determining the cellular composition of a tumor from a subject suffering from cancer, the method comprising the steps of:

- a) performing bulk mRNA sequencing on tumor material obtained from the subject in order to obtain bulk sequencing data,
- b) comparing the bulk mRNA sequencing data of step a) with a reference library comprising clustered sequencing data in order to determine the relative contribution of each cluster comprised in the clustered sequencing data to the bulk sequencing data obtained from the tumor material obtained from the subject, and
- c) determining the cellular composition of the tumor material obtained from the subject based on the relative contribution of each cluster comprised in the clustered sequencing data to the bulk sequencing data.

Claus 2. Method according to clause 1, wherein the step of comparing the bulk m RNA sequencing data with the reference library comprises using a tumor deconvolution algorithm on the bulk sequencing data.

Clause 3. Method according to clause 2, wherein said tumor deconvolution algorithm uses the reference library, and wherein the algorithm is used to calculate the relative contribution of each cluster in the reference library to the obtained bulk sequencing data.

Clause 4. Method according to any one of clauses 1 to 3, wherein both the bulk sequencing data and the reference library tumor materials are obtained from a tumor originating from or isolated from the same or comparable tissue types.

Clause 5. Method according to clause 4, wherein both the bulk sequencing data and the reference library are obtained from the same tumor type, preferably the tumor type being selected from the group consisting of breast cancer, melanoma, glioblastoma, lung cancer and colorectal cancer.

Clause 6. Method according to any one of clauses 1 to 5, wherein a prediction is made based on the cellular composition of tumor material obtained from the subject, preferably said prediction is based on a characteristic, comprising comparing the cellular composition of the tumor from the subject with at least one cellular composition of a comparison patient group with a known characteristic,

and wherein said prediction is preferably selected from the group consisting of:

- predicting progression free survival,
- predicting a treatment strategy,
- predicting a response to a treatment,
- linking cellular composition of a tumor to a treatment response,
- predicting recurrence free survival,
- predicting probability of metastasis formation, and

- predicting overall survival,

and wherein said prediction is based on comparing the cellular composition of the subject with the at least one cellular composition of the comparison patient group with known characteristic.

Clause 7. Method according to clause 6, wherein said characteristic is based on at least two comparison patient groups.

Clause 8. Method according to any one of clauses 1 to 6, wherein the reference library used in step b) is a library that was, independently, obtained by a method comprising the following steps:

- i) performing single-cell mRNA sequencing on individual cells isolated from tumor material obtained from at least one reference patient in order to obtain a sequencing data set for each sequenced cell,
- ii) generating a clustering of the sequenced single cells based on the sequencing data sets of step i) in order to obtain a reference library comprising clustered sequencing data sets.

Clause 9. Method according to clause 8, wherein the reference library was obtained by performing an additional step after step i) or after step ii), the step comprising:

- a) determining for each sequenced cell of step i) or each clustered data set of step ii) whether it is tumorigenic or non-tumorigenic.

Clause 10. Method according to clauses 8 or 9, wherein the reference library was obtained by single cell mRNA sequencing of at least 10 cells, preferably at least 100 cells, more preferably at least 1000 cells, most preferably at least 10000 cells, obtained from tumor material obtained from at least one reference patient.

Clause 11. Method according to any one of clauses 8 to 10, wherein the reference library was obtained by performing single cell mRNA sequencing on a plurality of tumor materials obtained from a plurality of reference patients and/or a plurality of tumor materials obtained from the same reference patient in step i). Clause 12. Method according to any one of clauses 8 to 11, wherein the reference library was obtained by a single-cell mRNA sequencing clustering algorithm in step ii).

Clause 13. Method according to any one of clauses 8 to 12, wherein the reference library was obtained by the clustering of step ii) being followed by an additional step of:

- iii) assigning to each cluster of sequencing data in the reference library a cell type or tumorigenic cell sub-type based on mRNA expression levels.

Clause 14. Method according to clause 13 wherein the reference library was obtained by performing an additional step of:

- iv) grouping the clustered sequencing data based on the assigned cell types and tumorigenic cell sub-types, and wherein all clustered sequencing data that were assigned the same cell type or same tumorigenic cell sub-type are grouped to form a group of clusters.

Clause 15. Method according to any one of clauses 11 to 14, wherein the tumors obtained from the reference patients are tumors originating from the same or comparable tissue types.

Clause 16. Method according to any one of clauses 11 to 15, wherein each tumor material in the reference library was obtained from the same tumor type, preferably wherein the tumor type is selected from the group consisting of breast cancer, melanoma, glioblastoma, lung cancer and colorectal cancer.

Clause 17. Method for preparing a reference library, the method comprising the following steps:

- i) performing single cell mRNA sequencing on individual cells isolated from tumor material obtained from at least one reference patient in order to obtain a sequencing data set for each sequenced cell,
- ii) generating a clustering of the sequenced single cells based on the sequencing data sets of step i) in order to obtain a reference library comprising clustered sequencing data sets.

Clause 18. Method according to clause 17, wherein step i) or step ii) is followed by an additional step of:

- a) determining for each sequenced cell of step i) or each clustered data set of step ii) whether it is tumorigenic or non-tumorigenic.

Clause 19. Method according to clause 18 wherein said single cell mRNA sequencing is performed on at least 10 cells, preferably at least 100 cells, more preferably at least 1000 cells, most preferably at least 10000 cells obtained from at least one reference patient.

Clause 20. Method according to any one of clauses 17 to 19, wherein said single cell mRNA sequencing in step i) is performed on a plurality of tumor materials obtained from a plurality of reference patients and/or obtained from a plurality of tumor materials obtained from the same patient.

Clause 21. Method according to any one of clauses 17 to 20, wherein said clustering in step ii) is a single-cell mRNA sequencing clustering algorithm.

Clause 22. Method according to any one of clauses 17 to 21, wherein the clustering is followed by an additional step of:

- iii) assigning to each cluster of sequencing data in the reference library a cell type or tumorigenic cell sub-type based on mRNA expression levels.

Clause 23. Method according to clause 22 wherein step iii) is followed by an additional step of:

- iv) grouping the clustered sequence data based on the assigned cell types and tumorigenic cell sub-type, and wherein all clustered sequencing data that were assigned the same cell type or same tumorigenic cell sub-type are grouped to form a group of clusters.

Clause 24. Method according to any one of cluases 20 to 23, wherein the tumors obtained from the reference patent are tumors originating from the same or comparable tissue types.

Clause 25. Method according to clause 24 wherein each of the tumor materials are obtained from the same tumor type, wherein the tumor type is selected from the group consisting of breast cancer, melanoma, glioblastoma, lung cancer and colorectal cancer.

Clause 26. Reference library obtainable by anyone of clauses 17 to 25.

**Claims**

1. Method for determining the cellular composition of a tumor from a subject suffering from cancer, the method comprising the steps of:

   - a) performing bulk mRNA sequencing on tumor material obtained from the subject in order to obtain bulk sequencing data,
   - b) comparing the bulk mRNA sequencing data of step a) with a reference library comprising clustered sequencing data in order to determine the relative contribution of each cluster comprised in the clustered sequencing data to the bulk sequencing data obtained from the tumor material obtained from the subject, and
   - c) determining the cellular composition of the tumor material obtained from the subject based on the relative contribution of each cluster comprised in the clustered sequencing data to the bulk sequencing data.

2. Method according to claim 1, wherein the step of comparing the bulk mRNA sequencing data with the reference library comprises using a tumor deconvolution algorithm on the bulk sequencing data.

3. Method according to claim 2, wherein said tumor deconvolution algorithm uses the reference library, and wherein the algorithm is used to calculate the relative contribution of each cluster in the reference library to the obtained bulk sequencing data.

4. Method according to any one of claims 1 to 3, wherein both the bulk sequencing data and the reference library tumor materials are obtained from a tumor originating from or isolated from the same or comparable tissue types, preferably wherein both the bulk sequencing data and the reference library are obtained from the same tumor type, preferably the tumor type being selected from the group consisting of breast cancer, melanoma, glioblastoma, lung cancer and colorectal cancer.

5. Method according to any one of claims 1 to 4, wherein a prediction is made based on the cellular composition of tumor material obtained from the subject, preferably said prediction is based on a characteristic, comprising comparing the cellular composition of the tumor from the subject with at least one cellular composition of a comparison patient group with a known characteristic, and wherein said prediction is preferably selected from the group consisting of:

- predicting progression free survival,
- predicting a treatment strategy,
- predicting a response to a treatment,
- linking cellular composition of a tumor to a treatment response,
- predicting recurrence free survival,
- predicting probability of metastasis formation, and
- predicting overall survival,

and wherein said prediction is based on comparing the cellular composition of the subject with the at least one cellular composition of the comparison patient group with known characteristic,
preferably wherein said characteristic is based on at least two comparison patient groups.

6. Method according to any one of claims 1 to 5, wherein the reference library used in step b) is a library that was, independently, obtained by a method comprising the following steps:

- i) performing single-cell mRNA sequencing on individual cells isolated from tumor material obtained from at least one reference patient in order to obtain a sequencing data set for each sequenced cell,
- ii) generating a clustering of the sequenced single cells based on the sequencing data sets of step i) in order to obtain a reference library comprising clustered sequencing data sets,

preferably wherein the reference library was obtained by performing an additional step after step i) or after step ii), the step comprising:

- a) determining for each sequenced cell of step i) or each clustered data set of step ii) whether it is tumorigenic or non-tumorigenic,

preferably wherein the reference library was obtained by single cell mRNA sequencing of at least 10 cells, preferably at least 100 cells, more preferably at least 1000 cells, most preferably at least 10000 cells, obtained from tumor material obtained from at least one reference patient.

7. Method according to claim 6, wherein the reference library was obtained by performing single cell mRNA sequencing on a plurality of tumor materials obtained from a plurality of reference patients and/or a plurality of tumor materials obtained from the same reference patient in step i),
preferably wherein the reference library was obtained by a single-cell mRNA sequencing clustering algorithm in step ii).

8. Method according to claim 6 or 7, wherein the reference library was obtained by the clustering of step ii) being followed by an additional step of:

- iii) assigning to each cluster of sequencing data in the reference library a cell type or tumorigenic cell sub-type based on mRNA expression levels,

preferably wherein the reference library was obtained by performing an additional step of:

- iv) grouping the clustered sequencing data based on the assigned cell types and tumorigenic cell sub-types, and wherein all clustered sequencing data that were assigned the same cell type or same tumorigenic cell sub-type are grouped to form a group of clusters.

9. Method according to claim 7 or 8, wherein the tumors obtained from the reference patients are tumors originating from the same or comparable tissue types, preferably wherein each tumor material in the reference library was obtained from the same tumor type, preferably wherein the tumor type is selected from the group consisting of breast cancer, melanoma, glioblastoma, lung cancer and colorectal cancer.

10. Method for preparing a reference library, the method comprising the following steps:

- i) performing single cell mRNA sequencing on individual cells isolated from tumor material obtained from at least one reference patient in order to obtain a sequencing data set for each sequenced cell,
- ii) generating a clustering of the sequenced single cells based on the sequencing data sets of step i) in order to obtain a reference library comprising clustered sequencing data sets.

11. Method according to claim 10, wherein step i) or step ii) is followed by an additional step of:

> - a) determining for each sequenced cell of step i) or each clustered data set of step ii) whether it is tumorigenic or non-tumorigenic,

> preferably wherein said single cell mRNA sequencing is performed on at least 10 cells, preferably at least 100 cells, more preferably at least 1000 cells, most preferably at least 10000 cells obtained from at least one reference patient.

12. Method according to claim 10 or 11, wherein said single cell mRNA sequencing in step i) is performed on a plurality of tumor materials obtained from a plurality of reference patients and/or obtained from a plurality of tumor materials obtained from the same patient,
preferably wherein said clustering in step ii) is a single-cell mRNA sequencing clustering algorithm.

13. Method according to any one of claims 10 to 12, wherein the clustering is followed by an additional step of:

> - iii) assigning to each cluster of sequencing data in the reference library a cell type or tumorigenic cell sub-type based on mRNA expression levels,

> preferably wherein step iii) is followed by an additional step of:

> - iv) grouping the clustered sequence data based on the assigned cell types and tumorigenic cell sub-type, and wherein all clustered sequencing data that were assigned the same cell type or same tumorigenic cell sub-type are grouped to form a group of clusters.

14. Method according to claim 12 or 13, wherein the tumors obtained from the reference patent are tumors originating from the same or comparable tissue types, preferably wherein each of the tumor materials are obtained from the same tumor type, wherein the tumor type is selected from the group consisting of breast cancer, melanoma, glioblastoma, lung cancer and colorectal cancer.

15. Reference library obtainable by anyone of claims 10 to 14.

Figure 1:

Figure 1

Figure 2:

Figure 3

Figure 4

Figure 5

Figure 6

Figure 7

Figure 8

Europäisches
Patentamt

European
Patent Office

Office européen
des brevets

**EUROPEAN SEARCH REPORT**

Application Number

EP 18 02 0578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/191553 A1 (MASSACHUSETTS EYE & EAR INFIRMARY [US] ET AL.) 18 October 2018 (2018-10-18) * the whole document * | 1-15 | INV. C12Q1/6809 C12Q1/6886 |
| X | LEI HAOYUN ET AL: "Tumor Copy Number Data Deconvolution Integrating Bulk and Single-cell Sequencing Data", 2018 IEEE 8TH INTERNATIONAL CONFERENCE ON COMPUTATIONAL ADVANCES IN BIO AND MEDICAL SCIENCES (ICCABS), IEEE, 18 October 2018 (2018-10-18), page 1, XP033451220, DOI: 10.1109/ICCABS.2018.8542078 [retrieved on 2018-11-20] * the whole document * | 1-15 | |
| X | WO 2017/004153 A1 (BROAD INST INC [US]; MASSACHUSETTS INST OF TECHNOLGY [US] ET AL.) 5 January 2017 (2017-01-05) * the whole document * | 1-15 | |
| X | SIDHARTH V. PURAM ET AL: "Single-Cell Transcriptomic Analysis of Primary and Metastatic Tumor Ecosystems in Head and Neck Cancer", CELL, vol. 171, no. 7, 14 December 2017 (2017-12-14), pages 1611-1624, XP055547353, AMSTERDAM, NL ISSN: 0092-8674, DOI: 10.1016/j.cell.2017.10.044 * the whole document * | 1-15 | TECHNICAL FIELDS SEARCHED (IPC) C12Q |

-/--

The present search report has been drawn up for all claims

| Place of search | Date of completion of the search | Examiner |
|---|---|---|
| The Hague | 30 April 2019 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 02 0578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | XURAN WANG ET AL: "Bulk tissue cell type deconvolution with multi-subject single-cell expression reference", NATURE COMMUNICATIONS, vol. 10, no. 1, 22 January 2019 (2019-01-22), XP55584261, DOI: 10.1038/s41467-018-08023-x * the whole document * | 1-15 | |

TECHNICAL FIELDS
SEARCHED     (IPC)

The present search report has been drawn up for all claims

| Place of search | Date of completion of the search | Examiner |
|---|---|---|
| The Hague | 30 April 2019 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

# EP 3 650 556 A1

## ANNEX TO THE EUROPEAN SEARCH REPORT
## ON EUROPEAN PATENT APPLICATION NO.

EP 18 02 0578

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2019

| Patent document cited in search report | Publication date | Patent family member(s) | Publication date |
|---|---|---|---|
| WO 2018191553 A1 | 18-10-2018 | NONE | |
| WO 2017004153 A1 | 05-01-2017 | EP 3314020 A1<br>US 2018100201 A1<br>WO 2017004153 A1 | 02-05-2018<br>12-04-2018<br>05-01-2017 |

EPO FORM P0459

For more details about this annex : see Official Journal of the European Patent Office, No. 12/82

37

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHU Y.** RNA sequencing: platform selection, experimental design, and data interpretation. *Nucleic Acid Ther.,* August 2012, vol. 22 (4), 271-4
- **GRÜN D ; MURARO MJ ; BOISSET JC et al.** De Novo Prediction of Stem Cell Identity using Single-Cell Transcriptome Data. *Cell stem cell,* 2016, vol. 19, 266-77
- **GRÜN D ; LYUBIMOVA A ; KESTER L et al.** Single-cell messenger RNA sequencing reveals rare intestinal cell types. *Nature,* 2015, vol. 525, 251-5
- **HRDLICKOVA R. et al.** RNA-Seq methods for transcriptome analysis. *Wiley Interdiscip Rev RNA,* January 2017, vol. 8 (1
- **KIM C ; GAO R ; SEI E et al.** Chemoresistance Evolution in Triple-Negative Breast Cancer Delineated by Single-Cell Sequencing. *Cell,* 2018
- **LI B ; DEWEY CN.** RSEM: accurate transcript quantification from RNA-Seq data with or without a reference genome. *BMC Bioinformatics,* 2011, vol. 12, 323
- **LI H. et al.** Reference component analysis of single-cell transcriptomes elucidates cellular heterogeneity in human colorectal tumors. *Nat Genet.,* May 2017, vol. 49 (5), 708-718
- **MURARO M. et al.** A Single-Cell Transcriptome Atlas of the Human Pancreas. *Cell Syst.,* 26 October 2016, vol. 3 (4), 385-394.e3
- **TIROSH I ; IZAR B ; PRAKADAN SM et al.** Dissecting the multicellular ecosystem of metastatic melanoma by single-cell RNA-seq. *Science,* 2016, vol. 352, 189-96
- **TIROSH I ; VENTEICHER AS ; HEBERT C et al.** Single-cell RNA-seq supports a developmental hierarchy in human oligodendroglioma. *Nature,* 2016, vol. 539, 309-13
- **PATEL AP ; TIROSH I ; TROMBETTA JJ et al.** Single-cell RNA-seq highlights intratumoral heterogeneity in primary glioblastoma. *Science,* 2014, vol. 344, 1396-401
- **QIU X. et al.** Reversed graph embedding resolves complex single-cell trajectories. *Nat Methods,* October 2017, vol. 14 (10), 979-982
- **SATIJA R. et al.** Spatial reconstruction of single-cell gene expression data. *Nat Biotechnol.,* May 2015, vol. 33 (5), 495-502
- **WANG Z. et al.** RNA-Seq: a revolutionary tool for transcriptomics. *Nat Rev Genet.,* January 2009, vol. 10 (1), 57-63
- **ZEISEL A. et al.** Brain structure. Cell types in the mouse cortex and hippocampus revealed by single-cell RNA-seq. *Science,* 06 March 2015, vol. 347 (6226), 1138-42